# EUROPÄISCHE PATENTSCHRIFT

(11) **EP 3 116 650 B1**
(45) Veröffentlichungstag und Bekanntmachung des Hinweises auf die Patenterteilung: **04.03.2020**
(21) Anmeldenummer: 15708206.6
(22) Anmeldetag: 05.03.2015
(51) Int. Cl.: G01N 21/64, G01N 33/543, G01N 21/03, B01L 3/00

(54) **VERFAHREN UND VORRICHTUNG ZUR BESTIMMUNG BIOLOGISCHER ANALYTEN**
METHOD AND DEVICE FOR THE DETERMINATION OF BIOLOGICAL ANALYTES
PROCÉDÉ ET DISPOSITIF DE DÉTERMINATION D'ANALYTES BIOLOGIQUES

(30) Priorität: 10.03.2014 EP 14158692
(43) Veröffentlichungstag der Anmeldung: 18.01.2017
(73) Patentinhaber: PAIA Biotech GmbH, 51105, Köln (DE)
(72) Erfinder: GIEHRING, Sebastian, 50678 Köln (DE)
(74) Vertreter: Zehetner Patentanwälte
(86) Internationale Anmeldenummer: PCT/EP2015/054671
(87) Internationale Veröffentlichungsnummer: WO 2015/135840

(56) Entgegenhaltungen:
- EP-A1- 0 371 265
- WO-A1-00/52451
- WO-A2-02/073198
- US-A- 5 674 699
- US-A1- 2005 214 167
- US-A1- 2007 154 356
- US-A1- 2009 251 683
- US-A1- 2010 028 935

## Beschreibung

Die Anmeldung betrifft ein Verfahren zur qualitativen und/oder quantitativen Bestimmung biologischer Analyten durch Messung der Lumineszenz der ungebundenen Lumineszenzmarker, sowie eine Vorrichtung hierfür und das Verwenden der Vorrichtung.

Bei der Entwicklung moderner Wirkstoffe, sonstiger Forschung im Bereich der Lebenswissenschaften und bei der Produktion von Biopharmazeutika stellt die quantitative und/oder qualitative Bestimmung und das Auszählen biologischer Einheiten wie z. B. Nukleinsäuren, Proteinen, Antikörper, Bakterien, Viren, oder Zellen, sogenannter biologischer Analyten, eine wichtige Methode dar.

Die Anforderungen an eine Methode oder Verfahren zur Bestimmung biologischer Analyten sind im Allgemeinen hoch und variieren sehr, insbesondere im Hinblick auf Konzentrationsbereiche, Messgenauigkeit und Probenvorbereitung. Selbst bei der Bestimmung derselben biologischen Analyten können die Messmethoden, abhängig vom Zweck der Messung, stark variieren. So wird beispielsweise in der Diagnostik ein Protein in einer sehr niedrigen Konzentration in Blutserumproben gemessen, während Proteine sich bei biotechnologischen Herstellungsverfahren in einem Kulturmedium befinden und dort in Konzentrationen vorliegen, die mehrere Zehnerpotenzen höher liegen. Ferner können die Methoden so beschaffen sein, dass sie neben der Bestimmung der Analyten noch weitere Informationen, z. B. über die Aktivität oder die Affinität und die Spezifität liefern können. Seit Jahrzehnten ist der sogenannte "enzyme linked immunoabsorbent assay", abgekürzt "ELISA-Assay" oder "ELISA-Verfahren" als die Standard-methode zur Bestimmung bestimmter biologischer Analyten, insbesondere Proteine und Antikörper etabliert. Hierbei handelt es sich um einen Immunoassay, an den eine enzymatische Farbreaktion gekoppelt ist. Das Prinzip des ELISA-Assays besteht im Wesentlichen darin, dass der biologische Analyt (z. B. ein Protein) durch Fängermoleküle an eine Oberfläche (z. B. eine Mikrotiterplatte) gebunden wird. Die Fängermoleküle sind typischerweise Antikörper, Fragmente davon oder auch Protein A oder Protein G.

Im weitverbreiteten sogenannten Sandwich-ELISA-Verfahren wird als Fänger ein Antikörper verwendet und anschließend ein Detektionsantikörper an einer zweiten Bindungsstelle an den Analyten gebunden. An den Detektionsantikörper ist ein Enzym gekoppelt (z. B. Meerrettichperoxidase, Horseradishperoxidase, HRP), welches durch Zugabe eines Substrats für das Enzym eine enzymatische Umsetzung auslöst, durch welche Farbstoffe entstehen. Typische Substrate sind Tetramethylbenzidin (TMB) oder Diaminobenzidin (DAB), die die Lösung blau oder braun färben. Bevor diese Färbung spektrometrisch gemessen werden kann, muss die enzymatische Reaktion durch Zugabe eines Stoppreagenzes gestoppt werden. Die durch das HRP produzierte Menge an Farbstoff ist proportional zur Menge an Analyt und wird dann mit einem geeigneten Detektionsgerät, in der Regel einem UV/VIS-Photometer, gemessen. Es sind in diesem Kontext auch Enzymsubstrate bekannt, die ein fluoreszierendes Produkt ergeben (z. B. Resorufin), das mit einem Fluoreszenzmessgerät detektiert wird. Durch die enzymatische Umsetzung kann im Sandwich-ELISA-Assay für jedes gebundene Analytmolekül eine große Anzahl an Farbstoffmolekülen produziert werden. Durch diesen Verstärkungseffekt ist der Sandwich-ELISA-Assay in der Regel sehr sensitiv bzw. nachweisstark.

Verbreitet sind auch ELISA-Verfahren, die dadurch gekennzeichnet sind, dass die Bindung des enzymkonjugierten Detektionsantikörpers nicht direkt an den Analyten erfolgt, sondern an einen weiteren, sogenannten primären Antikörper, der seinerseits spezifisch an den Analyten bindet. Diese Variante wird angewendet wenn keine enzymkonjugierten primären Antikörper verfügbar sind und ist insgesamt anfälliger für unspezifische Bindungen der Antikörper untereinander.

Die direkten ELISA-Verfahren zeichnen sich dadurch aus, dass kein Antikörper als Fängermolekül eingesetzt wird, sondern ein Antigen, welches durch den zu messenden Antikörper gebunden wird. Die Detektion verläuft analog zu den oben beschriebenen Verfahren über Detektionsantikörper.

Ein Nachteil des ELISA-Verfahrens ist, dass es eine Vielzahl von Inkubationsschritten und Waschschritten beinhaltet, die oftmals mehrmals durchgeführt werden müssen. Diese Arbeitsschritte sind nicht oder nur teilweise automatisierbar und werden deshalb derzeit überwiegend manuell durchgeführt. Das macht den ELISA-Assay personal- und zeitintensiv. Beim ELISA-Verfahren ergeben sich oft Versuchszeiten von 4 bis 5 Stunden, so dass der ELISA-Assay in Prozessen, bei denen ein schnelles Ergebnis benötigt oder eine Vielzahl an Proben gemessen werden soll, z. B. im HTS (High-Throughput-Screening), keine Anwendung hat.

Nachteilig am ELISA-Verfahren ist ferner, dass sich durch die Vielzahl an Arbeitsschritten, ein relativer großer Gesamtfehler ergibt, da jeder der Arbeitsschritte eine eigene Varianz verursacht und diese Varianzen sich zur Gesamtvarianz aufaddieren.

Es wurden bereits Versuche unternommen, das ELISA-Verfahren schneller und weniger personal- und kostenintensiv zu machen. Eine Abwandlung des ELISA-Verfahrens stellt der "Fluoreszenz-Immunoassay" dar (FIA oder "fluorescent immunoassay"). Fluoreszenz-Immunoassays sind in der Regel einfacher durchzuführen als das vorgenannte "klassische" ELISA-Verfahren.

Durch Verwendung eines mit einem Fluoreszenzfarbstoff markierten Detektionsantikörpers kann im Fluoreszenz-Immunoassay ohne enzymatische Reaktion gearbeitet werden. Dadurch reduziert sich die Anzahl der Verfahrensschritte, und Abweichungen, die auf schwankender Enzymaktivität beruhen, sind dadurch eliminiert. Diese Fluoreszenz-Immunoassays können in homogenen oder in heterogenen Systemen durchgeführt werden. In Letzteren werden oft spezielle Partikel eingesetzt und die Fluoreszenz an den Partikeln gemessen, während in homogenen Fluoreszenz-Immunoassays die Fluoreszenzmessung in Lösung erfolgt. Damit werden mehrere Schritte des klassischen ELISA-Verfahrens eingespart und die Versuchszeit auf gewöhnlich 2 bis 3 Stunden reduziert. Der Verzicht auf die Enzymreaktion hat aber oft zur Folge, dass die Sensitivität in diesen Methoden niedriger ist als im ELISA.

Ein Beispiel für einen homogenen Fluoreszenz-Immunoassay ist die Delfia-Technologie (Dissociation-Enhanced Lanthanide Fluorescent Immunoassay) der Firma Perkin-Elmer. In dem Verfahren werden an dem Detektionsantikörper gebundene Europium-Komplexe in Lösung gebracht und anschließend mit Fluoreszenz, insbesondere zeitaufgelöster Fluoreszenz, gemessen. Ein weiteres bekanntes Verfahren ist die TR-FRET-Technologie, die von der Firma Cisbio als HTRF-Technologie ("homogeneous time resolved fluorescence") angeboten wird, und die LANCE-Technologie ("Lanthanide Chelate Excite") der Firma Perkin-Elmer.

Vorgenannte TR-FRET Methode beruht auf dem Prinzip, dass zwei einander bindende Antikörper derart mit Fluoreszenzfarbstoffen markiert sind, dass diese einen Fluoreszenzresonanztransfer (FRET) durchführen können, wenn sie in räumliche Nähe gebracht werden, also wenn sie aneinander gebunden sind. An dem einen Antikörper ist ein FRET-Donor und an dem anderen Antikörper ein FRET-Akzeptor gebunden. FRET funktioniert nur dann, wenn der Abstand der Antikörper zueinander kleiner als 10 nm ist. Binden vorgenannte Antikörper aneinander, kann der FRET-Donor mit Licht einer bestimmten Wellenlänge zur Fluoreszenz angeregt werden. Durch das Emissionslicht des FRET-Donors wird der FRET-Akzeptor zur Fluoreszenz-Emission angeregt und diese gemessen. D. h. dass nur dann die Fluoreszenz-Emission des FRET-Akzeptors gemessen werden kann, wenn beide Antikörper (in der Lösung) aneinander gebunden sind. Wird nun eine solche Lösung vorgelegt und ein Analyt dazugegeben, der an den FRET-Akzeptor oder FRET-Donor bindet, wird einer der beiden FRET-Partner aus dem Komplex verdrängt, so dass sich die Fluoreszenz-Emission des FRET-Akzeptors in Abhängigkeit von der Konzentration des Analyten verringert.

FRET ist eine in der Forschung weit verbreitete Technik um die Bindung von zwei Molekülen nachzuweisen. Es ist aber auch bekannt, dass die Effizienz des Transfers stark von der Probenzusammensetzung abhängt. Um diese Technik zu einem routinefähigen Verfahren zu machen, werden in der TR-FRET-Technologie spezielle FRET-Donoren eingesetzt, die aus einem Komplex zwischen einem Europium- oder Terbiumion und einem Makrozkylus (z. B. auf der Basis von Trisbipyridinen) bestehen. Diese haben die Eigenschaft, dass sie im Vergleich zu anderen fluoreszierenden Stoffen, die sich in der Probe befinden können, langlebigere Fluoreszenz ausstrahlen. Die Fluoreszenzmessung findet daher erst in einem definierten zeitlichen Abstand nach einem Lichtblitz statt. Dadurch wird abgewartet, bis die Störfluoreszenz aus der Probe abgeklungen ist und nur noch die FRET-Fluoreszenz auftritt. Dennoch müssen bei der TR-FRET-Technologie sowohl die Emission des FRET-Donors als auch die des FRET-Akzeptors gemessen werden und ihr Verhältnis bestimmt werden um Matrixeinflüsse und Quenching (Auslöschung) der Fluoreszenz-Emission rechnerisch zu kompensieren. Damit diese Kompensation optimal funktioniert müssen beide Emissionswellenlängen gleichzeitig und nicht sequentiell gemessen werden. Das bedeutet, dass die Messgeräte für genaue TR-FRET-Messungen mit zwei Detektoren ausgerüstet sein müssen. Typische Messbereiche für das TR-FRET-Verfahren liegen bei ca. 10 - 5000 ng/mL Antikörper.

Eine weitere Abwandlung des klassischen ELISA-Assays stellt das AlphaLISA-Verfahren von Perkin-Elmer dar. Hier wird ein mit Sonden markiertes Paar von Antikörpern durch Bindung an unterschiedliche Bindungsstellen desselben Analyten in räumliche Nähe gebracht.

Im Gegensatz zum TR-FRET werden hierbei keine Fluorophore mit kleinem Molekulargewicht als Sonden benutzt, sondern Donor- und Akzeptorpartikel (sogenannte Donor- und Akzeptorbeads) mit einer Größe von ca. 250 - 350 nm verwendet. Durch Bestrahlung der Donorbeads mit Laserlicht einer Wellenlänge von 680 nm wird von diesen Singulettsauerstoff freigesetzt, der an dem in der Nähe befindlichen Akzeptorbead eine Lichtemission einer Wellenlänge von ca. 615 nm auslöst. Diese wird anschließend gemessen und dient der Quantifizierung der Analyten. Anders als im TR-FRET können im AlphaLISA die sondentragenden Moleküle nicht gleichzeitig zugegeben werden, so dass eine zweite Reaktionszeit (Inkubationszeit) erforderlich ist und sich das Protokoll konsequenterweise verlängert.

Die Donorbeads sind lichtempfindlich. Mit ihnen kann nur im Dunklen oder in Grünlicht gearbeitet werden, was einen erheblichen Nachteil der Methode darstellt. Der im AlphaLISA Assay zu verwendende Laser ist oft nur in spezielle Fluoreszenzplattenreader eingebaut, was den Versuch teuer macht, da man die Geräte haben oder kaufen muss.

Bei partikelgestützten heterogenen Fluoreszenz-Immunoassays werden, wie in den klassischen ELISA-Verfahren, zunächst der biologische Analyt durch Fängermoleküle an die Oberfläche, in diesem Fall an funktionalisierte nichtlösliche Teilchen, sogenannte Partikel, gebunden. Nach einem Waschschritt, werden fluoreszenzmarkierte Detektionsantikörper hinzugegeben, vermengt und anschließend werden die ungebundenen fluoreszenzmarkierten Detektionsantikörper entfernt. Gemessen wird die Menge der an die Partikel gebundenen fluoreszenzmarkierten Detektionsantikörper, wobei der Gehalt des biologischen Analyten über die Fluoreszenz der auf den Partikeln gebundenen Fluoreszenzmarker bestimmt wird. Eine solche Methode bzw. Bestimmung ist bekannt (siehe Übersichtsartikel von C. F. Woolley und M. A. Hayes, "Recent developments in emerging microimmunoassays", Bioanalysis (2013) 5(2)).

Bei der von der Firma Gyros AB eingesetzten "GYROS-Technologie" werden mikrofluidische Strukturen eingesetzt, um kleine Probenmengen auf Partikelschüttungen (gewöhnlich in Form kleiner Säulen) zu zentrifugieren, auf denen fluoreszenzmarkierte Sandwichkomplexe entstehen, die gemessen werden. Die gesamte Mikrofluidik wird in runden Plastikscheiben realisiert, wobei die Flüssigkeiten (z. B. gelöste Fängermoleküle, Probe, Waschlösungen) durch die Drehung der Scheibe (wie bei einer CD-Rom) um ihre Mittelachse nach außen durch die Kanäle gedrückt werden. Da mit der GYROS-Technologie nur Konzentrationen von grösser gleich 1ng/mL gemessen werden können, wird sie vor allem in der biotechnologischen Prozessentwicklung und -kontrolle eingesetzt.

Die Firma Quanterix bietet eine Technologie namens SIMOA ("Single molecule array") an, bei der Sandwich-Immunokomplexe an Partikeln gebildet werden. Anschließend werden die Partikel auf einem Chip mikrofluidisch auf hunderttausende Kavitäten (Vertiefungen/ Ausbuchtungen), die jeweils nur einen Partikel aufnehmen können, verteilt. Für die Detektion werden die Kavitäten dann verschlossen und ein Enzym aktiviert, das einen Fluoreszenzfarbstoff erzeugt. Bei sehr niedrigen Konzentrationen an Analyt befindet sich im Schnitt weniger als ein Analytmolekül in einer Kavität, d. h. es gibt nur Kavitäten mit oder ohne Fluoreszenz, die dann gezählt werden. Dieser Assay wird daher auch als "digitaler ELISA" bezeichnet. Damit können Detektionsgrenzen unter 10⁻¹⁵ M erreicht werden (siehe Rissin et al.: "Single-molecule enzyme-liked immunosorbent assays detects serum proteins at subfemtomolar concentrations", Nature Biotechnology, 28(6), 595).

GYROS und SIMOA sind mehrstufige Verfahren und man benötigt aufwendige mikrostrukturierte Verbrauchsmittel (wie z. B. die o. g. Scheiben) sowie dedizierte und teure Auslesegeräte.

Zur Messung von fluoreszenzmarkierten Immunokomplexen, die an Partikel gebunden sind, können auch Durchflusszytometer verwendet werden. Hier werden die Partikel durch ein Flusssystem einzeln an einen Messpunkt transportiert, an dem sie mit Laserlicht zur Fluoreszenz angeregt werden. Die Messung mit einem Durchflusszytometer hat den Vorteil, dass man unterschiedliche Analyten gleichzeitig bestimmen kann. Hierzu werden Partikel eingesetzt, die in ihrem Inneren eine Farbcodierung tragen, d. h. mit unterscheidbaren Fluoreszenzfarbstoffen dotiert sind, wobei jede der Farben für eine Bindung an einen bestimmten Analyten steht. Zur Messung werden zwei Laser gleichzeitig eingesetzt. Der erste Laser identifiziert die Partikelsorte über die Farbcodierung und der zweite Laser den Gehalt an gebundenem Immunokomplex. In der XMAP-Technologie (Luminex Corp.) können in einer Versuchsdurchführung bis zu hundert verschiedene Analyten detektiert werden; hier spricht man von Multiplexanwendungen.

Neben vorgenannten Verfahren werden, insbesondere im Bereich der Prozessentwicklung und - kontrolle zur Herstellung von rekombinanten Proteinen und Antikörpern, weitere Messverfahren verwendet, die weniger komplex als ELISA sind, z. B. die Biolayer Interferometry (BLI, von Pall ForteBio) oder die Oberflächenplasmonenresonanz (Surface Plasmon Resonance SPR, GE Healthcare, Biacore). Diese Verfahren, insbesondere die BLI, erlauben einen höheren Automatisierungsgrad und basieren auf der Bindung des Analyten an Oberflächen, die typischerweise mit Protein A oder G oder mit Antikörpern beschichtet sind.

Die Bindung der Analyten an die Oberfläche bewirkt eine Veränderung der optischen Eigenschaft der Oberfläche, welche gemessen wird. Daraus wird die Menge der an die Oberfläche gebundenen Moleküle ermittelt. So werden bei BLI Messbereiche von 0,5 - 2000 µg/mL und bei der SPR Messbereiche von 0,15 -10 µg/mL erreicht. Ein Nachteil dieser Methode besteht darin, dass vergleichsweise teure und spezialisierte Messgeräte benötigt werden, sowie spezielle Verbrauchsmittel (z. B. Sensoren und Chips).

Alle die vorgenannten Verfahren sind im Hinblick auf ihre komplexe Versuchsdurchführung nachteilig. Sie benötigen oft viele Arbeitsschritte für die Quantifizierung biologischer Analyten und sind daher aufwändig durchzuführen. Gleichfalls gibt es Anwendungen und wissenschaftliche Fragestellungen, in denen es wünschenswert ist, ein vereinfachtes, auf einem Immunoassay basierendes, Verfahren zur Verfügung zu haben, mit dem höher konzentrierte Proben gemessen werden können und/oder mit dem man schnell Ergebnisse erzielt. Weiterhin ist es wünschenswert einen Immuoassay zur Verfügung zu haben, mit dem spezielle Antikörper in diversen Flüssigkeiten, wie z. B. Serum oder Zellkulturüberständen, bestimmt werden können. Das ist insbesondere bei der rekombinanten (technischen) Herstellung von Antikörpern von Vorteil, um während der Herstellung von Proteinen einfach, schnell und kostengünstig qualitative und/oder quantitative Aussagen machen zu können. Ferner ist es wünschenswert, ein Verfahren, insbesondere einen Immunoassay, zur Bestimmung biologischer Analyten zur Verfügung zu haben, das mit wenigen Arbeitsschritten auskommt, wodurch Zeit und Reagenzien gespart werden können. Es besteht deshalb ein Bedürfnis nach einem einfachen, leicht durchführbaren und schnellen Verfahren zur Bestimmung biologischer Analyten, vorzugsweise als Immunoassay, das die vorgenannten Nachteile ganz oder zumindest teilweise vermeidet.

Aufgabe der vorliegenden Erfindung ist es deshalb, ein Verfahren bereitzustellen, mit dem vorgenannte Nachteile ganz oder zumindest teilweise vermieden werden.

Die Aufgabe wurde mit dem in den Ansprüchen beschriebenen Verfahren, insbesondere durch die erfindungsgemäße Vorrichtung bzw. deren Verwendung gelöst. Entscheidend ist das Messen der Lumineszenz- bzw. Fluoreszenz-Emission der ungebundenen Lumineszenz- bzw. Fluoreszenzmarker, wodurch die Bestimmung biologischer Analyten einfach, schnell und kostengünstig durchgeführt werden kann.

Die Erfindung betrifft deshalb ein Verfahren zur Bestimmung (vor allem zur Quantifizierung) von biologischen Analyten in einer wässrigen Lösung in Gegenwart einer oder mehrerer funktionalisierter Oberflächen, wobei die wässrige Lösung mindestens eine Art von biologischen Analyten und mindestens eine Art Lumineszenzmarker, bevorzugt Fluoreszenzmarker, enthält, dadurch gekennzeichnet, dass zur Bestimmung der Menge und/oder Konzentration des oder der biologischen Analyten die Lumineszenz-Emission, bevorzugt Fluoreszenz-Emission, der ungebundenen Lumineszenzmarker, bevorzugt Fluoreszenzmarker, gemessen wird.

Mit dem erfindungsgemäßen Verfahren können beliebige Assays durchgeführt werden. Bevorzugt handelt es sich beim erfindungsgemäßen Verfahren um sogenannte Immunoassays, insbesondere um solche, die ausgewählt sind aus einer Gruppe bestehend aus direkten Immunoassay, Sandwich Immunoassay, Verdrängungs- oder Displacement-Immunoassay (hierin auch Inhibitionsassay genannt), kompetitiven Immunoassay und sekundären Immunoassay.

Es sei darauf hingewiesen, dass alle unten angegebenen Ausführungsformen, die nicht vom Schutzbereich der in den beiliegenden Patentansprüchen definierten Erfindung erfasst sind, nicht als Ausführungsformen und Ausführungsbeispiele der Erfindung anzusehen sind.

In einer Ausführungsform [V-1] werden im erfindungsgemäßen Verfahren als funktionalisierte Oberflächen funktionalisierte Partikel aus Polymer oder einem Polymergemisch verwendet, auf deren Oberfläche Fängermoleküle vorhanden sind, die mit dem oder den biologischen Analyten und/oder mit dem oder den Fluoreszenzmarkern eine Bindung eingehen. Die funktionalisierten Partikel weisen vorteilhafterweise einen mittleren Durchmesser im Bereich von etwa 20 bis etwa 200 µm oder im Bereich von etwa 80 bis etwa 200 µm auf.

In einer nicht-erfindungsgemäßen Ausführungsform [V-2] werden im erfindungsgemäßen Verfahren als funktionalisierte Oberflächen funktionalisierte magnetische Partikel verwendet. Die magnetischen Partikel weisen vorteilhafterweise einen mittleren Durchmesser im Bereich von etwa 1 bis etwa 100 µm auf. Magnetische Partikel weisen ferromagnetische oder superparamagnetische Eigenschaften auf. Sie enthalten üblicherweise einen magnetischen Kern aus Ferrit oder Magnetit, der von einer Schale aus Polymeren oder Polymergemischen umschlossen ist. Sie sind entsprechend der nicht-magnetischen Partikel funktionalisiert, d. h. mit Fängermolekülen ausgestattet.

In einer Ausführungsform [V-3] umfasst das erfindungsgemäße Verfahren folgende Schritte:
(a) Einbringen mindestens einer Art funktionalisierter Partikel in eine Messkammer, die einen durch den Boden der Messkammer lichtzugänglichen Detektionsbereich und einen lichtunzugänglichen Abtrennbereich aufweist;
(b) Einbringen einer Probe enthaltend mindestens eine Art biologischer Analyt in die Messkammer;
(c) Einbringen mindestens einer Art Fluoreszenzmarker in die Messkammer;
   (c') Vermischen der eingebrachten Partikel, Probe und Fluoreszenzmarker in der Messkammer;
   (c") Trennung der ungebundenen Fluoreszenzmarker von gebundenen Fluoreszenzmarkern (vorzugsweise durch Sedimentation oder Zentrifugation), so dass sich die gebundenen Fluoreszenzmarker im Abtrennbereich befinden;
(d) Messen der Fluoreszenz-Emission der ungebundenen Fluoreszenzmarker im Detektionsbereich; und
(e) Bestimmen der Menge und/oder Konzentration des oder der biologischen Analyten.

Unter Messkammer wird hier auch ein erfindungsgemäß ausgestattetes Well einer Mikrotiterplatte verstanden. Das erfindungsgemäße Verfahren wird bevorzugt mit Mikrotiterplatten durchgeführt, deren Wells als erfindungsgemäße Messkammern ausgestattet sind.

Die Schritte (a), (b) und (c) können vor oder nach einem weiteren Verfahrensschritt durchgeführt werden, wobei der weitere Verfahrensschritt das Einbringen von weiteren Versuchskomponenten oder Substanzen, wie z. B. primäre Antikörper oder Hilfsstoffe, wie Detergenzien, in die Messkammer umfasst.

Die Schritte (a), (b) und (c) können sequentiell erfolgen oder mindestens zwei der Schritte erfolgen gleichzeitig.

In einer Variante [V-4] des erfindungsgemäßen Verfahrens werden Schritt (a) und/oder Schritt (c) vor den übrigen Verfahrensschritten durchgeführt, vorzugsweise in einen großen zeitlichen Abstand zu den übrigen Schritten.

In einer nicht-erfindungsgemäßen Variante [V-5] des Verfahrens sind die funktionalisierten Partikel magnetisch (magnetisierbar).

In einer weiteren nicht-erfindungsgemäßen Variante [V-6] werden funktionalisierte magnetische Partikel verwendet und Schritt (c") erfolgt durch Sedimentation und durch Anlegen eines temporären oder permanenten Magnetfelds.

In einer Ausführungsform [V-7] des erfindungsgemäßen Verfahrens wird zur Messung der Fluoreszenz-Emission der ungebundenen Fluoreszenzmarker in Schritt (d) ein Fluoreszenzmikroskop verwendet. Bei den zu verwendenden erfindungsgemäßen Vorrichtungen, Messkammern oder Mikrotiterplatten kann dann die lichtundurchlässige Schicht am Boden entfallen. Das Fluoreszenzmikroskop muss so eingestellt sein, dass es nur die auftretende Fluoreszenz-Emission im Detektionsbereich misst, während die aus dem Abtrennbereich kommende Fluoreszenz-Emission ausgeblendet ist.

Mikrotiterplatten sind bekannt. Sie enthalten eine Vielzahl voneinander isolierter Kavitäten (auch Wells, Näpfchen, Vertiefungen oder Mulden genannt). Die Anzahl an Kavitäten auf einer Mikrotiterplatte kann variieren. Käuflich erhältlich sind folgende Anordnungen:
2x3 (6 Wells), 4x3 (12 Wells), 4x6 (24 Wells), 6x8 (48 Wells), 8x12 (96 Wells), 16x24 (384 Wells), 32x48 (1536 Wells). Der Boden in den Näpfchen der käuflich erhältlichen Mikrotiterplatten kann unterschiedlich geformt sein. Käuflich erhältlich sind folgende Böden: F-Boden (Flachboden), C-Boden (Flachboden mit minimal abgerundeten Ecken), V-Boden (konisch zulaufender Boden) und U-Boden (U-förmige Vertiefung). Erfindungsgemäß geeignet sind insbesondere Mikrotiterplatten mit einem F-, C oder U-Boden in die die Vorrichtung eingebracht wird.

Partikel-gestützte Verfahren zur Bestimmung von biologischen Analyten sind bekannt (siehe US 4,731,337B, DE 102004038163A, WO 86/04684, WO 94/29722, US 4,115,535B, WO 2011/045022). Diese Verfahren basieren alle darauf, dass die Fluoreszenz von gebundenen Fluoreszenzmarkern gemessen wird.

Vorrichtungen zum Abtrennen von unlöslichen Bestandteilen aus wässrigen Lösungen sind bekannt. So beschreiben beispielsweise WO 2011/031236 und US 2010/0028935 speziell geformte Vorrichtungen zum Abtrennen von korpuskularen Partikeln aus einer wässrigen Lösung, um damit die Messgenauigkeit bei der Transmissionsspektroskopie bzw. Absorptionsspektroskopie zu erhöhen. Diese Vorrichtungen sind jedoch nicht für die Verwendung in dem erfindungsgemäßen Verfahren geeignet, da es mit diesen Vorrichtungen nicht möglich ist, die Lumineszenz ungebundener Lumineszenzmarker, bzw. die Fluoreszenz ungebundener Fluoreszenzmarker verlässlich zu messen, um den oder die zu bestimmenden biologischen Analyten zu quantifizieren.

Deshalb sind auch spezielle Vorrichtungen Gegenstand des Verfahrens, mit denen das erfindungsgemäße Verfahren durchgeführt werden kann.

Somit betrifft die Erfindung auch eine Vorrichtung [0] zur Bestimmung biologischer Analyten durch Messen der Lumineszenz der ungebundenen Lumineszenzmarker, in der gebundene und ungebundene Lumineszenzmarker in einer wässrigen Lösung unter Verwendung einer oder mehrerer funktionalisierter Oberflächen räumlich und optisch voneinander getrennt sind, wobei die Vorrichtung für die Trennung ein mindestens teilweise lichtdurchlässiges Strukturelement aufweist, wobei die Basis der Vorrichtung bis auf die Grundfläche des Strukturelements lichtundurchlässig ist und die wässrige Lösung mindestens eine Art von biologischen Analyten und mindestens eine Art Lumineszenzmarker enthält, und wobei mindestens eine Art von biologischen Analyten und ggf. mindestens eine Art von Lumineszenzmarkern an eine oder mehrere funktionalisierte Oberflächen bindet.

Eine nicht-erfindungsgemäße Ausführungsform betrifft eine Messkammer zur Verwendung im erfindungsgemäßen Verfahren, in dem funktionalisierte magnetische Partikel eingesetzt werden. Die Trennung der gebundenen von den ungebundenen Lumineszenzmarkern, bevorzugt Fluoreszenzmarkern, wird durch gerichtete Sedimentation mit Hilfe eines unter der Messkammer platziertem lichtundurchlässigen Magnetelement bewirkt. Das Magnetelement weist Aussparungen auf, die so angeordnet sind, dass am Boden (Basis) der Messkammer Messfenster entstehen. Das Magnetelement kann fest mit der Basis verbunden sein oder es ist abnehmbar. Die Messkammer weist kein Strukturelement auf.

Durch das Magnetfeld wird die Sedimentation der funktionalisierten magnetischen Partikel so gelenkt, dass sich oberhalb des Messfensters keine Partikel absetzen. Die Messung der Lumineszenz, bevorzugt Fluoreszenz, erfolgt durch Messung mit einem Fluoreszenzmessgerät durch das Messfenster am Boden der Messkammer.

Eine nicht-erfindungsgemäße Ausführungsform betrifft weiterhin die Verwendung der vorgenannten Messkammer bzw. einer Mikrotiterplatte, die mit mindestens einer solchen Messkammer ausgestattet ist, im erfindungsgemäßen Verfahren, wobei gleichfalls funktionelle magnetische Partikel verwendet werden.

In einer Ausführungsform [A] der erfindungsgemäßen Vorrichtung [0] werden eine oder mehrere funktionalisierte Oberflächen als funktionalisierte Partikel in die erfindungsgemäße Vorrichtung eingebracht, wobei die gebundenen Lumineszenzmarker sich im Abtrennbereich der Vorrichtung und die ungebundenen Lumineszenzmarker sich im Detektionsbereich befinden, wobei sie dort vorzugsweise homogen verteilt sind.

In einer Ausführungsform [B] der erfindungsgemäßen Vorrichtung [0] befinden sich innerhalb der Vorrichtung eine oder mehrere funktionalisierte Oberflächen, vorzugsweise im Bereich der Vorrichtung, der sich unterhalb des der Basis abgewandten Endes des Strukturelements befindet, vorzugsweise der sich auf der Basis der Verrichtung befindet. Die Trennung erfolgt, indem der biologische Analyt und der Lumineszenzmarker mit einer oder mehreren der sich in der Vorrichtung befindenden, ggf. immobilisierten, funktionalisierten Oberflächen eine Bindung eingehen.

In einer weiteren Ausführungsform [C] betrifft die Erfindung eine Vorrichtung, wie oben oder in der Ausführungsform [A] oder [B] beschrieben, in der das Strukturelement als Erhebung (vorzugsweise als eine sich nach oben verjüngende Erhebung) ausgestaltet ist, deren Querschnitt eine beliebige Geometrie aufweisen kann (z. B. kreisförmig, eckig, dreieckig), wobei das von der Basis der Vorrichtung abgewandte Ende des Strukturelements so beschaffen ist, dass sich dort keine Versuchskomponenten, insbesondere keine Partikel ablagern. Es kann z. B. flach sein oder eine konvexe Form einnehmen und/oder einen geringen Durchmesser aufweisen.

Das Strukturelement, das sich in einer Messkammer befindet, kann die Form eines Dorns, Kegels, abgeschnittenen Kegels, einer Pyramide oder abgeschnittenen Pyramide deren Grundfläche n-eckig ist, aufweisen, wobei n für eine ganze Zahl im Bereich von 3 bis 10 steht, vorzugsweise für 3, 4, 5, 6, 7 oder 8. Es kann ferner eine Form haben, die von einem Kegel oder einer Pyramide abgeleitet ist, z. B. Kegel auf rundem Sockel oder 4-seitige Pyramide auf einem quadratischen Sockel.

Das Strukturelement kann ferner am von der Basis der Vorrichtung abgewandten Ende ein optisches Bauteil z. B. eine Linse aufweisen.

Das Strukturelement ist lichtdurchlässig. Es besteht aus einem geeigneten lichtdurchlässigen Material oder Materialgemisch. Wichtig ist, dass das Material keine die Messung störende Autofluoreszenz aufweist. Lichtdurchlässige Materialien sind bekannt und können vom Fachmann ohne Weiteres ausgewählt werden. Solche Materialien sind z. B. Polystyrol, COC (Cycloolefin-Copolymer), Polypropylen oder Polymethylmethacrylat (PMMA). Gut geeignet sind Polypropylen und PMMA. Polystyrol, insbesondere wenn es heiß verarbeitet wird, weist ein gewisses Maß an Autofluoreszenz auf (Young et al, Anal Chem. 2013 January 2; 85(1): 44-49) und ist deshalb erfindungsgemäß nicht bevorzugt.

Das Material oder Materialgemisch kann in einer Stärke (Schichtdicke) von ≤1 mm verwendet werden. Bevorzugte Stärken liegen in folgenden Bereichen: von etwa 0,05 bis etwa 0,5 mm, von etwa 0,1 bis etwa 0,45 mm, von etwa 0,15 bis etwa 0,4 mm, von etwa 0,2 bis etwa 0,4 mm oder von etwa 0,2 bis etwa 0,35 mm.

In einer weiteren Ausführungsform [D] betrifft die Erfindung eine Vorrichtung, wie oben oder in einer der Ausführungsformen [A], [B] oder [C] beschrieben, in der es einen Abtrennbereich und einen Messbereich (Detektionsbereich) gibt, wobei der Messbereich ein lichtdurchlässiges Messfenster aufweist oder mit einem solchen optisch verbunden ist.

In einer weiteren Ausführungsform [E] betrifft die Erfindung eine Vorrichtung, wie oben oder in einer der Ausführungsformen [A], [B], [C] oder [D] beschrieben, in der die Basis des Strukturelements als Messfenster ausgestaltet ist. Die Messung der Lumineszenz erfolgt dann mit Hilfe eines Lumineszenz-, vorzugsweise Fluoreszenzmessgeräts. Die Anregung und die Detektion der Emission erfolgen von unterhalb der Vorrichtung.

Wenn die Basis und das Ende des Strukturelements, das sich in einer Vorrichtung bzw. Messkammer befindet, optisch miteinander verbunden sind, gelangt Anregungslicht, das von der Basis der Vorrichtung her eingestrahlt wird, durch das Strukturelement hindurch in den Detektionsbereich und regt dort die ungebundenen Lumineszenzmarker, bevorzugt Fluoreszenzmarker, zur Emission an, die dann gleichfalls von der Basis der Vorrichtung gemessen wird. Das Ende des Strukturelements und die Basis bilden dann zusammen das Messfenster. Bei der Auswahl einer geeigneten Form des Strukturelements ist zu berücksichtigen, dass das Anregungs- und Emissionslicht nicht von Partikeln gestört wird. Besonders vorteilhaft ist es deshalb, wenn das Ende des Strukturelements eine konvexe Form einnimmt und das Strukturelement als Erhebung ausgestaltet ist, die sich nach oben verjüngt. Ist das Ende flach, so ist es vorteilhaft, wenn die Fläche klein ist und/oder die Fläche so beschaffen ist, dass sich dort keine oder nur eine nicht-störende Anzahl an Partikel (vorliegend auch als "im wesentlichen keine" bezeichnet) ablagern kann. Auch bei flachen Enden ist es vorteilhaft, wenn sich die Erhebung in Richtung des von der Basis abgewandten Endes verjüngt.

In einer weiteren Ausführungsform [F] betrifft die Erfindung eine Mikrotiterplatte, in deren Mulden, Näpfchen bzw. Vertiefungen jeweils eine Vorrichtung, wie oben oder in einer der Ausführungsformen [0], [A], [B], [C], [D] oder [E] beschrieben, integriert ist.

Entsprechend betrifft die Erfindung in einer Ausführungsform [F-1] eine Mikrotiterplatte zur Durchführung des erfindungsgemäßen Verfahrens, dadurch gekennzeichnet, dass in mindestens eine Vertiefung der Mikrotiterplatte eine Vorrichtung eingebracht ist, die ein mindestens teilweise lichtdurchlässiges Strukturelement aufweist, das als eine sich nach oben verjüngende Erhebung ausgestaltet ist und deren Querschnitt eine beliebige Geometrie aufweisen kann, wobei das von der Basis abgewandte Ende des Strukturelements so beschaffen ist, dass sich dort im wesentlichen keine Versuchskomponenten ablagern, und wobei die Basis der Vorrichtung bis auf die Grundfläche des Strukturelements lichtundurchlässig ist, und wobei die Ränder der Vertiefung die Seitenränder einer Messkammer bilden.

In einer weiteren Ausführungsform [F-2] betrifft die Erfindung die Mikrotiterplatte, wie in Ausführungsform [F-1] beschrieben, dadurch gekennzeichnet, dass der ursprüngliche Boden der Mikrotiterplatte gegen eine einstückige, Strukturelemente aufweisende Basis ersetzt ist, wobei sich in jeder der Vertiefungen der Mikrotiterplatte ein Strukturelement befindet, und wobei die Basis bis auf die Grundfläche der Strukturelemente lichtundurchlässig beschichtet ist.

In diesem Fall besteht die Basis aus dem gleichen Material wie die Strukturelemente und für die Auswahl des Materials, aus dem die Basis gebildet wird, gelten die im Zusammenhang mit den Strukturelementen genannten Kriterien bezüglich Materialeigenschaften und Schichtdicke.

Die Erfindung bezieht sich auch auf die Herstellung der als Ausführungsform [F-2] beschriebenen Mikrotiterplatte, umfassend die folgenden Schritte:
(x) Ersetzen des Bodens einer Mikrotiterplatte durch eine Strukturelemente aufweisende Basis, die vorzugsweise so viele Strukturelemente aufweist, wie in der Mikrotiterplatte Wells vorhanden sind;
(y) Verbinden der Basis mit der Mikrotiterplatte; und
(z) Aufbringen einer lichtundurchlässigen Schicht auf die Unterseite der Basis, wobei die Grundfläche des Strukturelements ausgespart wird.

Das Verbinden der Basis mit der Mikrotiterplatte und das Aufbringen einer lichtundurchlässigen Schicht erfolgen wie weiter unten im gleichen Kontext beschrieben.

Die einstückige Strukturelemente aufweisende Basis kann durch formgebende Verfahren hergestellt werden, z. B. durch Spritzgussverfahren, additive Herstellungsverfahren (z. B. 3D-Druck) oder Thermoformen (Warmformen, Tiefziehen oder Vakuumformen).

Es ist selbstverständlich möglich, dass sich nicht in jeder der Vertiefungen einer Mikrotiterplatte ein Strukturelement befindet. Gleichfalls ist es möglich, dass in den Vertiefungen der Mikrotiterplatte unterschiedlich geformte Strukturelemente vorhanden sind. Eine einstückige Strukturelemente aufweisende Basis kann somit unterschiedlich geformte Strukturelemente aufweisen.

In einer weiteren Ausführungsform [G] betrifft die Erfindung eine Messkammer, enthaltend eine Vorrichtung wie oben oder in einer der Ausführungsformen [0], [A], [B], [C], [D] oder [E] beschrieben.

In einer weiteren Ausführungsform [H] betrifft die Erfindung das Verwenden der Vorrichtung, Messkammer oder Mikrotiterplatte, wie oben oder in einer der Ausführungsformen [0], [A], [B], [C], [D], [E], [F] oder [G] beschrieben, in einem Immunoassay, insbesondere direktem Immunoassay, Sandwich Immunoassay, kompetitiven Immunoassay oder sekundären Immuno-assay, wobei dann die Lumineszenzmarker bevorzugt Fluoreszenzmarker sind, zur qualitativen oder quantitativen Bestimmung biologischer Analyten über die Messung der Lumineszenz der ungebundenen Lumineszenzmarker.

Neben oben beschriebenen Verfahren betrifft die Erfindung ferner ein Verfahren zur qualitativen und/oder quantitativen Bestimmung biologischer Analyten, unter Verwendung der erfindungsgemäßen Vorrichtung, Messkammer oder Mikrotiterplatte, insbesondere wie oben oder in einer der Ausführungsformen [0], [A], [B], [C], [D], [E], [F] oder [G] beschrieben, umfassend die folgenden Schritte:
(a) Einbringen mindestens einer Art funktionalisierter Oberflächen in die Vorrichtung;
(b) Einbringen einer Probe enthaltend mindestens eine Art biologischer Analyten in die Vorrichtung;
(c) Einbringen mindestens einer Art Lumineszenzmarker in die Vorrichtung;
(d) Messen der Lumineszenz-Emission der ungebundenen Lumineszenzmarker; und
(e) Bestimmen der Menge und/oder Konzentration der biologischen Analyten.

Erfindungsgemäß funktionalisierte Oberflächen können bereits in der Vorrichtung vorhanden sein oder sie werden beim erfindungsgemäßen Verfahren allein oder zusammen mit der Messlösung (Probe) in die Vorrichtung eingebracht.

In einer Ausführungsform der Erfindung werden als funktionalisierte Oberflächen funktionalisierte Partikel verwendet.

Im erfindungsgemäßen Verfahren können mehrere Arten funktionalisierter Oberflächen / Partikel, bzw. auf einer Oberfläche / Partikeloberfläche mehrere Arten an Funktionalisierung vorhanden sein. Das heißt, die Art der Funktionalisierung der Oberfläche kann unterschiedlich sein. Das hat zur Folge, dass mit dem erfindungsgemäßen Verfahren und in der erfindungsgemäßen Vorrichtung und Messkammer bzw. mit der erfindungsgemäßen Mikrotiterplatte mehrere Arten biologischer Analyten gemessen werden können. Dazu muss dann für jeden der Analyten ein eigener, mit unterschiedlicher Wellenlänge anzuregender und/oder emittierender Lumineszenzmarker verwendet werden.

Der Begriff "funktionalisiert" bedeutet im Zusammenhang mit der vorliegenden Erfindung, dass Fängermoleküle (hiernach auch Fänger genannt) auf einer Oberfläche, vorzugsweise auf einer Partikeloberfläche, vorhanden sind. Zur Funktionalisierung von Oberflächen bzw. Partikeloberflächen wird die Oberfläche mit mindestens einer Art von Fängermolekül belegt. Fängermoleküle sind Moleküle, die mit dem oder den biologischen Analyten und/oder mit dem oder den Lumineszenzmarkern eine physikalische oder chemische Bindung eingehen. Dabei ist es irrelevant, ob es sich um magnetische oder nicht-magnetische Partikel handelt. Beide Arten von Partikeln können wie hier beschrieben funktionalisiert werden.

Erfindungsgemäß bedeutet "funktionalisieren" auch das Beladen von magnetischen oder nicht-magnetischen Partikeln mit geeigneten Metallionen, wie weiter unten im Zusammenhang mit der immobilisierten Metallaffinitätschromatographie (IMAC) beschrieben, so dass hier die gebildeten Metallionen-Komplexe (z. B. NTA-Ni²⁺) die Fängermoleküle bilden, die dann solche Versuchskomponenten binden, die die entsprechenden Tags aufweisen.

Für kompetitive Assays ist es nötig, dass die funktionalisierten Oberflächen, insbesondere Partikeloberflächen, Fänger aufweisen, an denen sowohl der biologische Analyt als auch der Lumineszenzmarker an selbe oder gleiche Bindungsstelle physikalisch und/oder chemisch binden kann. Für die übrigen Assays sollten die Fänger so beschaffen sein, dass sie nur den oder die biologischen Analyten bzw. falls gewünscht, den oder die Lumineszenzmarker, vorzugsweise Fluoreszenzmarker, binden.

In einer Funktionalisierungsvariante verfügt das Fängermolekül über nur spezifisch für eine Art von biologischen Analyten ausgewählte Bindungsstellen, während in einer anderen Variante das Fängermolekül über mehrere unterschiedliche Bindungsstellen verfügt, wobei jede Bindungsstelle so ausgewählt ist, dass diese nur einen speziellen biologischen Analyten bindet. Mit dieser Variante ist es möglich, dass verschiedene Arten von biologischen Analyten an die funktionalisierte Oberfläche binden.

Sollen verschiedene Arten von biologischen Analyten gleichzeitig gemessen werden, so können im erfindungsgemäßen Verfahren auch verschieden funktionalisierte Partikel in einer Messkammer verwendet werden.

Eine Funktionalisierungsvariante umfasst das Ausstatten der Oberflächen mit unterschiedlichen Arten von Fängermolekülen, wobei jedes dieser Fängermoleküle mindestens eine Bindungsstelle für den zu bestimmenden biologischen Analyten aufweist.

Werden im erfindungsgemäßen Verfahren funktionalisierte Partikel verwendet, die mehrere Arten von biologischen Analyten zu binden vermögen, dann muss für jeden der Analyten ein eigener, mit unterschiedlicher Wellenlänge anzuregender und/oder emittierender Lumineszenzmarker, vorzugsweise Fluoreszenzmarker, verwendet werden.

Für die verlässliche Durchführung des erfindungsgemäßen Verfahrens, insbesondere wenn es sich um einen direkten (Immuno)Assay, Sandwich (Immuno)Assay oder sekundären (Immuno)Assay handelt ist es wichtig, dass weder die Fängermoleküle noch die Oberflächen bzw. Partikeloberflächen spezifische oder unspezifische Bindungen mit anderen Versuchskomponenten als den oder die biologischen Analyten eingehen. Ausnahmen hiervon stellen der kompetitive (Immuno)Assay und der Verdrängungsassay dar. Im kompetitiven (Immuno)Assay kann der Fänger an der gleichen Bindungsstelle den Lumineszenzmarker und den spezifischen biologischen Analyten binden. Im Verdrängungsassay bindet der Fänger den Lumineszenzmarker, vorzugsweise den Fluoreszenzmarker.

Zur Funktionalisierung von Oberflächen und Partikeloberflächen geeignete Fänger sind bekannt und werden so ausgewählt, dass die gewünschte Versuchskomponente oder die gewünschten Versuchskomponenten an den Fänger binden. Geeignete Fänger sind meistens Proteine (z. B. Antikörper), die gegebenenfalls mit Linkersystemen an die Oberflächen bzw. Partikeloberflächen gebunden werden. Die Belegung der Oberfläche bzw. Partikeloberfläche mit Fängern kann durch kovalente oder nicht-kovalente Bindung geschehen. Ist eine der Versuchskomponenten (z. B. der biologische Analyt) ein Antikörper, so ist der Fänger gewöhnlich ein Protein. Bekannte Fänger für Antikörper sind z. B. Protein A aus *Staphylococcus aureus* und Protein G aus Streptokokken.

Beispiele für mit einem Linkersystem ausgestattete Oberflächen sind Streptavidin beschichtete Partikel wie sie unten näher beschrieben sind, z. B. Streptavidin Mag-Sepharose® Partikel (VWR, Art.-Nr. 28-9857-38). Ein weiteres Beispiel sind Agarosepartikel die auf ihrer Oberfläche Nitriloessigsäure- (NTA) oder Iminoessigsäuregruppen (IDA) tragen, die mit Metallionen sehr stabile Komplexe bilden und die geeignet sind, mit dem sogenannten His-Tag markierte Proteine zu binden.

Ein gängiges, zur Herstellung funktionalisierter Partikel geeignetes Material, das aus porösem Material besteht ist Agarose, das unter dem Namen Sepharose™ (GE Healthcare) käuflich erhältlich ist. Sepharose bzw. Agarose ist in verschiedenen Vernetzungsgraden und mit unterschiedlichen Bindungskapazitäten erhältlich. Vor Funktionalisierung, d. h. dem Beladen mit Fängern, werden die Agarose-Partikel, mit einer Chemikalie aktiviert, um so eine effiziente Bindung mit Fängermolekülen zu gewährleisten. Eine zur Aktivierung geeignete Chemikalie ist CNBr.

Wie bereits beschrieben, sind gängige Fängermoleküle Proteine. Um Proteine an Oberflächen, insbesondere Partikeloberflächen zu binden, können Chemikalien verwendet werden, die als Linker zwischen der Oberfläche und dem Fängermolekül fungieren (z. B. Streptavidin oder dessen Homologe (z. B. Avidin, Neutravidin)). Streptavidin oder dessen Homologe und Biotin gehen eine stabile Bindung ein, wodurch jedes biotinylierte Protein an mit Streptavidin behandelte (Partikel)Oberflächen bindet.

Die Biotinylierung von Proteinen ist bekannt und eine Standardmethode in der Biochemie. Gewöhnlich wird zur Biotinylierung ein Aktivester (z. B. NHS, N-Hydroxysuccinimid) benutzt, mit dem Biotin an freie Aminofunktionen eines Fänger-Proteins gekoppelt wird. Biotinylierte Fänger-Proteine werden gewöhnlich an mit Streptavidin oder mit dessen Homologen behandelte Partikel gebunden.

Fänger-Proteine, die Protein-Affinitäts-Tags beinhalten, können über Metallkomplexe an eine (Partikel)Oberfläche gebunden werden. Hierzu bedient man sich im Wesentlichen dem Prinzip, dass gewisse Aminosäuren mit geeigneten Metallionen (z. B. Co²⁺, Ni²⁺, Cu²⁺, und Zn²⁺) stabile Komplexe bilden. Solche Aminosäuren können nativ im Protein vorhanden oder sie können als Polypeptide in das Protein, als sogenannte "Tags", eingeführt sein. Gängige Tags sind Arg-Tag, c-Myc-Tag, Flag-Tag und His-Tag. Die Metallionen werden mit Hilfe von NTA (Nitriloessigsäure), CMA (Carboxymethylaspartate) oder IDA (Iminoessigsäure) an die (Partikel)Oberfläche gebunden. Im Wesentlichen benutzt man bei der vorgenannten Funktionalisierung die gleichen Prinzipien, die man sonst bei der chromatographischen Aufreinigung von Proteinen, insbesondere bei der immobilisierten Metallaffinitätschromatographie (IMAC), anwendet.

Durch Ausnutzung vorgenannter Prinzipien, kann der Fachmann ohne Weiteres das erfindungsgemäße Verfahren an seine jeweiligen Bedürfnisse anpassen, indem die funktionalisierten Partikel individuell an den zu verwendenden Assay oder den oder die zu bestimmenden biologischen Analyten angepasst werden können.

Im Falle, dass die funktionalisierten Oberflächen bereits in der Vorrichtung vorhanden sind, bezieht sich die Erfindung in einer Ausführungsform darauf, dass das Einbringen der funktionalisierten Oberflächen in die Vorrichtung, d. h. Schritt (a), als Schritt (a') erfolgt, nämlich Behandeln mindestens einer Oberfläche innerhalb der Vorrichtung, vorzugsweise der Basis der Vorrichtung, wobei das der Basis abgewandte Ende des Strukturelements nicht behandelt wird, mit einem die Bindung der Fänger an die Basis begünstigenden Coating-Puffer und Zugeben von mindestens einer Art von Fänger, wobei die Fänger vorzugsweise in Lösung zugegeben werden.

Die Erfindung bezieht sich demzufolge auch auf eine Vorrichtung, erfindungsgemäße Messkammer und eine erfindungsgemäße Mikrotiterplatte, in der mindestens eine Oberfläche der Vorrichtung, vorzugsweise die Basis der Vorrichtung funktionalisiert ist, wobei das der Basis abgewandte Ende des Strukturelements nicht funktionalisiert ist. Behandelte Oberflächen einer Vorrichtung werden hier auch als "stationäre" Oberflächen bezeichnet.

Nach Zugabe der Fänger ist es vorteilhaft, wenn der Coating-Puffer an dem gewünschten zur Funktionalisierung vorgesehenen Ort mehrere Stunden oder über Nacht einwirkt. Für das Belegen von Oberflächen mit Fängermolekülen geeignete Coating-Puffer sind bekannt. Beispiel hierfür ist ein basischer Carbonat-Puffer, mit dem beispielsweise das Fängermolekül Protein A an eine gewünschte Stelle z. B. auf der Basis der Vorrichtung gebunden werden kann.

Ebenso kann die Funktionalisierung der Basis der Vorrichtung, der erfindungsgemäßen Messkammer und der erfindungsgemäßen Mikrotiterplatte, durch Streptavidin erfolgen, wodurch eine Vielzahl unterschiedlicher Fänger, die vorher jeweils mit Biotingruppen versehen (konjugiert) worden sind, an die gewünschte Stelle gebunden wird.

Durch die Auswahl geeigneter funktionalisierter Oberflächen kann Einfluss auf die Versuchsdauer genommen werden. Im Prinzip gilt, dass durch eine große funktionalisierte Oberfläche die Wahrscheinlichkeit erhöht wird, dass der oder die zu messenden biologischen Analyten und, sofern gewünscht und vorgesehen der oder die Lumineszenzmarker, aufgrund Diffusion auf Fänger treffen. Durch die Auswahl der geeigneten Größe der Oberfläche und der Art und Anzahl der darauf vorhandenen Fänger kann die Geschwindigkeit der Messung beeinflusst werden. Zur Beschleunigung des erfindungsgemäßen Verfahrens werden bevorzugt große Oberflächen verwendet, auf denen sich eine Vielzahl Fänger befinden. Das gilt für Partikeloberflächen genauso wie für stationäre Oberflächen.

Einen weiteren Faktor, der Einfluss auf die Geschwindigkeit des erfindungsgemäßen Verfahrens hat, stellt, neben der Größe der verwendeten funktionalisierten Oberfläche, deren Bindungskapazität dar. Bei funktionalisierten Partikeln wird unter deren Bindungskapazität verstanden: Kapazität der Bindung einer bestimmten Menge an biologischen Analyt auf dem funktionalisierten Partikel in Bezug auf die Menge bzw. Gewicht, Masse an vorhandenen funktionalisierten Partikeln. Ein weiterer geschwindigkeitsbestimmender Parameter im erfindungsgemäßen Verfahren ist, wie unten genauer beschrieben, das sorgfältige Vermischen der Versuchskomponenten. Dabei ist es unerheblich, ob es sich um magnetische oder nicht-magnetische Partikel handelt.

Die im erfindungsgemäßen Verfahren als funktionalisierte Oberflächen zu verwendenden funktionalisierten Partikel sind käuflich erhältlich oder werden für das Verfahren entsprechend individuell hergestellt.

Zur Herstellung solcher individuell funktionalisierter Partikel können insbesondere Partikel aus synthetischen oder natürlichen Polymeren, z. B. Polystyrol, Latex, Agarose, Polylactid oder PMMA sowie Polysaccharide, eingesetzt werden. Partikel aus porösen Materialien, wie z. B. Agarose, sind bevorzugt. Insbesondere eignen sich käuflich erhältliche Partikel, die zur chromatographischen Aufreinigung von biologischen Analyten, z. B. Proteinen, eingesetzt werden. Sie werden gewöhnlich als Aufschlämmung (Slurry) angeboten. Vorgenanntes gilt analog für magnetische Partikel, wobei die synthetischen oder natürlichen Polymere den Magnetkern umschließen.

Die erfindungsgemäß verwendbaren nicht-magnetischen Partikel sind oft sphärische Partikel und weisen typische mittlere Durchmesser im Bereich von etwa 0,1 bis etwa 200 µm auf. Erfindungsgemäß bevorzugte Partikel weisen mittlere Durchmesser im Bereich von etwa 20 bis etwa 200 µm, insbesondere von etwa 80 bis etwa 200 µm auf und gehen keine unspezifischen Bindungen mit den Versuchskomponenten ein. Partikel, die eine Bindungskapazität von mehr als 20 mg IgG bzw. Analyt pro mL aufweisen, sind erfindungsgemäß bevorzugt.

Die nicht-erfindungsgemäßen magnetischen Partikel (auch "magnetische beads" genannt) sind solche, die gewöhnlich für die Isolierung oder Analyse von biologischen Analyten verwendet werden (siehe z. B. WO 2006/112771). Die Polymerbeschichtung von magnetischen Partikel besteht gewöhnlich aus Zuckern, Polyvinylalkohol oder aus Silikaten. Die magnetischen Partikel sind in analoger Weise, wie für die Partikel beschrieben, funktionalisierbar oder sind bereits als funktionalisierte magnetische Partikel käuflich erhältlich (z. B. Protein A Mag-Sepharose® Partikel von GE Healthcare Art.-Nr. 28-9440-06). Erfindungsgemäß besonders geeignete magnetische Partikel besitzen eine Beschichtung aus porösem Material z. B. Agarose und haben einen Kern aus Ferrit oder Magnetit (z. B. Mag-Sepharose® Partikel).

Magnetische Partikel sind gleichfalls sphärische Partikel und weisen typische mittlere Durchmesser im Bereich von etwa 1 bis etwa 100 µm auf.

Die in Kombination mit einem bestimmten biologischen Analyten zu verwendenden funktionalisierten nicht-magnetischen Partikel sind vom Fachmann leicht zu ermitteln und teilweise werden solche Kombinationen bereits in den weiterentwickelten Immunoassay-Verfahren des Standes der Technik verwendet.

Die Schritte (a) (ggf. (a')), (b) und (c) können im erfindungsgemäßen Verfahren nacheinander, d. h. sequentiell durchgeführt werden oder mindestens zwei der Schritte (a), (b) und (c) erfolgen gleichzeitig, z. B. durch Vorlegen von funktionalisierten Oberflächen in der Vorrichtung, gefolgt von der Messlösung, die die Probe und die Lumineszenzmarker enthält. Alternativ wird die Probe enthaltend den Analyten vorgelegt und der Lumineszenzmarker und die funktionalisierte Oberfläche in Form funktionalisierter Partikel dazu gegeben. Bei der sequentiellen Zugabe kommt es gewöhnlich nicht auf die Reihenfolge der Schritte an. Es ist möglich Schritt (b) vor Schritt (a) und (c), oder Schritt (b) vor Schritt (c) und (a), oder Schritt (c) vor Schritt (a) bzw. (b) durchzuführen. Bei kompetitiven Immunoassays ist es vorteilhaft, wenn Schritt (b) und Schritt (c) gleichzeitig durchgeführt werden. Wird eine Vorrichtung verwendet, die bereits funktionalisierte Oberflächen aufweist, so entfällt Schritt (a).

Bevorzugt werden im erfindungsgemäßen Verfahren Schritt (a) und Schritt (c), gleichzeitig oder sequentiell, vor den übrigen Verfahrensschritten durchgeführt. Gleichfalls bevorzugt wird Schritt (a) oder Schritt (c) vor den übrigen Verfahrensschritten durchgeführt.

Dabei kann der zeitliche Abstand zwischen den Schritten (a) und/oder (c) und den übrigen Versuchsschritten gering oder groß sein (z. B. einige Stunden, Tage, Wochen, Monate oder Jahre). Ist der Abstand größer als 12 Stunden, so ist es vorteilhaft, wenn die in den Schritten (a) und/oder (c) eingebrachten Versuchskomponenten, d. h. die eingebrachten funktionalisierten Partikel und/oder Fluoreszenzmarker in der Messkammer getrocknet vorliegen. Die Messkammern werden bei Raumtemperatur, bevorzugt bei einer erhöhten Temperatur von maximal 37 °C, bevorzugt 35 °C, getrocknet. Die Dauer der Trocknung richtet sich gewöhnlich an die Menge der in der Messkammer eingebrachten wässrigen Lösung. Übliche Trocknungszeiten liegen im Bereich von 12 bis 24 Stunden.

Vor Trocknung können gegebenenfalls weitere Substanzen in die Messkammer eingebracht werden, die dafür sorgen sollen, dass die Funktionalität/Aktivität der Partikel und/oder der Lumineszenzmarker bzw. Fluoreszenzmarker nach Trocknung erhalten bleibt. Geeignete Substanzen sind BSA, Zucker oder Detergenzien wie Tween 20.

Die Erfindung betrifft deshalb auch die erfindungsgemäßen Messkammern und Mikrotiterplatten in denen
(i) mindestens eine Art von funktionalisiertem Partikel und mindestens eine Art von Fluoreszenzmarker, oder
(ii) mindestens eine Art von funktionalisiertem Partikel oder
(iii) mindestens eine Art von Fluoreszenzmarker,
jeweils in getrocknetem Zustand vorliegen, sowie deren Verwendung im erfindungsgemäßen Verfahren.

Die Erfindung betrifft gleichfalls deren Herstellung. Hierzu wird wie oben beschrieben vorgegangen, nämlich durch Trocknung oder Gefriertrocknung der entsprechenden Versuchskomponente nach Einbringen in die erfindungsgemäße Messkammer.

Werden Messkammern verwendet, in denen bereits mindestens eine Art von funktionalisiertem Partikel und/oder mindestens eine Art von Fluoreszenzmarker, in getrocknetem Zustand vorliegen, so entfällt im erfindungsgemäßen Verfahren der oder die Schritte, mit denen die betreffende Versuchskomponente zugegeben wird. Z. B. liegen die funktionalisierten Partikel bereits getrocknet vor, so entfällt das Einbringen von funktionalisierten Partikel, d. h. Schritt (a).

Im erfindungsgemäßen Verfahren bilden sich zwischen den Versuchskomponenten Bindungen aus, die von den Affinitäten der Versuchskomponenten zueinander und deren Konzentrationen abhängen. Für das erfindungsgemäße Verfahren ist es erforderlich, dass die Menge an ungebundenen Lumineszenzmarkern bei gegebenen und konstanten Versuchsbedingungen nur von der Menge an biologischem Analyten abhängt.

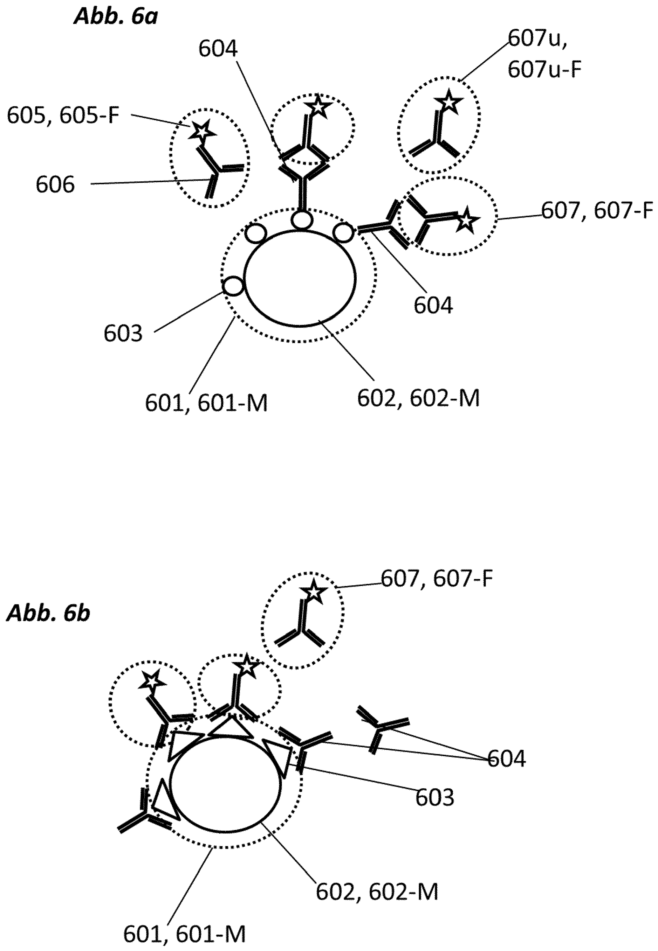

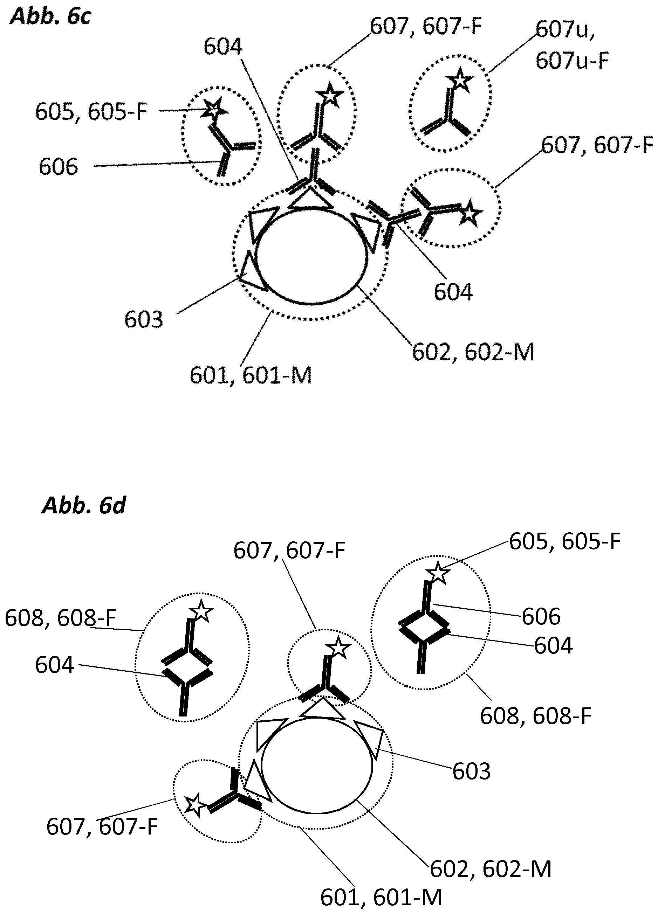

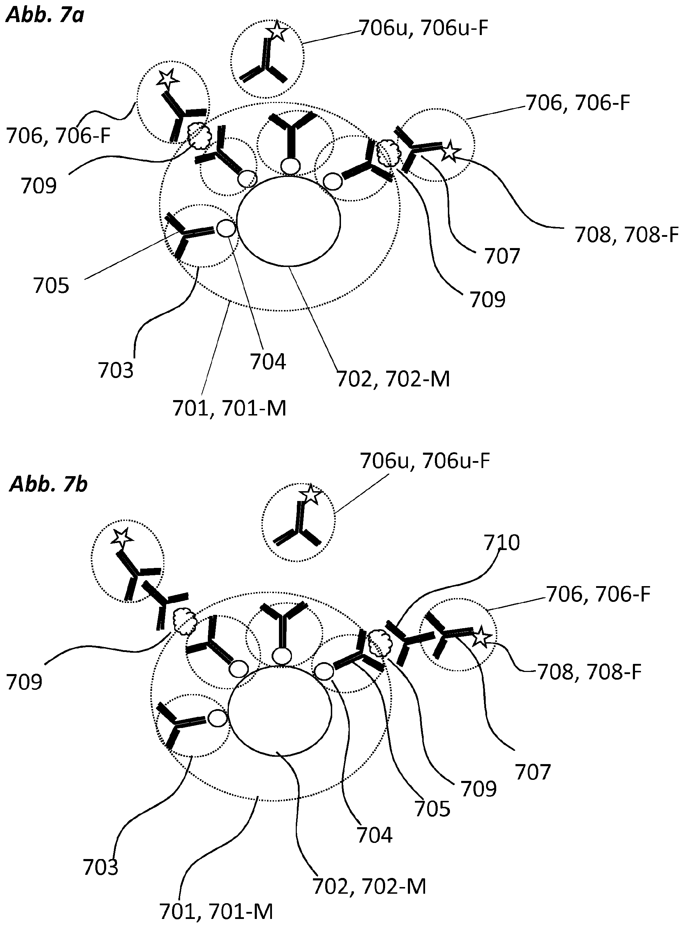

In einer Ausführungsform der Erfindung gehen der Lumineszenzmarker und der Fänger an unterschiedlichen Stellen (Epitopen) mit dem biologischen Analyt jeweils eine Bindung ein (schematisch dargestellt in Abbildungen 6a, 6c und 7a).

In einer weiteren Ausführungsform der Erfindung können der Lumineszenzmarker und der biologische Analyt jeweils an derselben Stelle am Fängermolekül binden (schematisch dargestellt in Abbildung 6b).

In einer weiteren Ausführungsform der Erfindung bindet ein sogenannter "primärer Antikörper", anstelle des Lumineszenzmarkers an den biologischen Analyten und der Lumineszenzmarker bindet an den primären Antikörper (schematisch dargestellt in Abbildung 7b)

Um das Auftreten einer Bindung zu beschleunigen, ist es sinnvoll, die Versuchskomponenten nach dem Einbringen aller Versuchskomponenten in die erfindungsgemäße Vorrichtung, Messkammer oder Mikrotiterplatte gut zu vermischen. Das kann manuell oder automatisiert erfolgen. Je schneller die Versuchskomponenten in Kontakt kommen, desto schneller verläuft die Einstellung des Gleichgewichts und somit die Messung/Bestimmung des biologischen Analyten.

Wird eine schnellere Durchführung des Verfahrens gewünscht, kann auf die Einstellung des Gleichgewichts verzichtet werden. In diesem Fall ist es wichtig, für alle Proben und Kalibratoren gleiche Inkubationszeiten und Bedingungen einzuhalten.

Für das automatisierte Vermischen wird die Einheit, in der sich die erfindungsgemäße Vorrichtung befindet (z. B. Messkammer oder Mikrotiterplatte), auf laborübliche Schüttler (Schüttelgeräte) gestellt. Solche Schüttelgeräte sind bekannt. Die optimale Schüttelgeschwindigkeit kann mit einfachen Methoden herausgefunden werden. Auch das Verwenden einer Vortex-Apparatur ist denkbar. Ziel des Vermischens ist es, die Auftreffwahrscheinlichkeit der Versuchskomponenten aufeinander zu erhöhen, so dass sich die gewünschten Bindungen möglichst zügig ausbilden und daher die Gleichgewichtseinstellung der Bindung der Komponenten schnell erreicht wird. Die Zeit für die Gleichgewichtseinstellung der Bindung der Komponenten kann variieren und leicht herausgefunden werden. Gewöhnlich werden bei optimalen Durchmischung Zeiten im Bereich von 15 bis 30 Minuten zu einer für die Messung ausreichenden Gleichgewichtseinstellung der Bindung der Komponenten benötigt.

Alternativ zur freien Diffusion oder dem Schütteln, kann die Auftreffwahrscheinlichkeit der Versuchskomponenten aufeinander, insbesondere des biologischen Analyten auf ein Fängermolekül, erhöht werden, wenn die funktionalisierte Oberflächen (vorzugsweise in Form von funktionalisierter Partikel) in Fluidiksysteme oder Mikrofluidiksysteme eingebracht werden. In diesen werden sie aktiv von dem oder den zu bestimmenden biologischen Analyten angespült, wodurch es zu einer Anreicherung des oder der biologischen Analyten auf derfunktionalisierten Oberfläche (vorzugsweise funktionalisierten Partikel) kommt. Mikrofluidische Systeme werden oft zur Erhöhung der Sensitivität der Detektion in schwierigen Proben, z. B. humanem Serum verwendet.

Erfindungsgemäße Lumineszenzmarker können die gewünschten Versuchskomponenten (insbesondere den oder die biologischen Analyten) binden und gleichzeitig sind sie in der Lage Licht zu emittieren. In der erfindungsgemäßen Vorrichtung und in dem erfindungsgemäßen Verfahren können alle denkbaren Lumineszenzmarker eingesetzt werden. Bevorzugt werden Fluoreszenzmarker eingesetzt.

Im Rahmen der vorliegenden Erfindung werden unter dem Begriff "gebundener Lumineszenzmarker" alle Lumineszenzmarker verstanden, die direkt oder über weitere Versuchskomponenten an die funktionalisierten Oberflächen gebunden sind (z. B. via Analyt oder einen primären Antikörper). Gleichzeitig bezieht sich der Begriff "ungebundener" Lumineszenz- oder Fluoreszenzmarker auf solche, die weder direkt noch über weitere Versuchskomponenten an die funktionalisierte Oberfläche gebunden sind.

Lumineszenz- bzw. Fluoreszenzmarker, die an bestimmte biologische Analyten und/oder funktionalisierte Oberflächen binden, sind bekannt und sind käuflich erhältlich. Geeignet sind beispielsweise Alexa 647- oder Alexa 488-konjugierte Antikörperfragmente (z. B. Alexa 647-konjugiertes AffiniPure F(ab')₂ Fragment Goat Anti-Human IgG F(ab')₂ specific und polyklonaler Kaninchen anti-Huhn IgY (H+L)-Alexa Fluor488, Alexa 488 AffiniPure F(ab')₂ Fragment GoatAnti-Human IgG, F(ab')₂ specific) oder Fluorescein-Isothiocyanat (FITC)-konjugierter polyklonaler Huhn anti-human IgG (H+L) Antikörper und R-Phycoerythrin AffiniPure F(ab')₂ Fragment Goat Anti-Human IgG, Fcγ Fragment specific.

Lumineszenz bzw. Fluoreszenzmarker können auch individuell hergestellt werden. Hierzu wird ein Lumineszenz- bzw. Fluoreszenzfarbstoff mit einem biologischen Molekül (z. B. Protein, Antikörper, Antikörperfragment), das eine geeignete Bindungsstelle aufweist, und die gewünschte Versuchskomponente (bevorzugt den oder die zu bestimmenden biologischen Analyten) binden kann, verknüpft. Die Verknüpfung erfolgt nach gängigen Methoden, z. B. durch Verwenden von Lumineszenz- bzw. Fluoreszenzfarbstoffen, die mit NHS- Ester- oder Maleimidengruppen ausgestattet sind und so an Antikörper und Proteine gekoppelt werden können. Die Verknüpfung kann außerdem, ähnlich wie oben beschrieben, Streptavidin vermittelt sein, indem ein biotinyliertes Protein an einen mit Streptavidin ausgestatteten Lumineszenz- bzw. Fluoreszenzfarbstoff gebunden wird. Die natürlichen fluoreszierenden Proteine, wie z. B. GFP (Green Fluorescent Protein) und seine Derivate können entweder alleine oder zusammen mit anderen Proteinen, als sogenannte Fusionsproteine, als Fluoreszenzmarker verwendet werden.

Die Auswahl des Farbstoffes und der Bindungsstelle erfolgt in Abhängigkeit vom dem durch das Verwenden der erfindungsgemäßen Vorrichtung bzw. Durchführen des erfindungsgemäßen Verfahrens zu bestimmenden biologischen Analyten und dem dafür zu verwendenden Assay.

Es können alle bekannten Fluoreszenzfarbstoffe verwendet werden, die auch sonst bei Messungen biologischer Proben zum Einsatz kommen. Solche Farbstoffe sind z. B. Cyanine, Cumarine, Fluoresceine, Rhodamine sowie deren Derivate, wie z. B. Fluorescin-Isothiocyanat. Sie sind käuflich erhältlich (z. B. Alexa™, Dy-Light™, Atto™ oder Oyster™).

Neben vorgenannten Fluoreszenzfarbstoffen können auch fluoreszierende Quantenpunkte ("Qdots") zur Herstellung von Fluoreszenzmarkern verwendet werden. Solche Fluoreszenzmarker zeichnen sich durch eine besonders hohe Fluoreszenz-Emission aus.

Es ist vorstellbar, dass der zu bestimmende biologische Analyt selbst mit einem Fluoreszenzfarbstoff gekoppelt und als Fluoreszenzmarker benutzt wird. Solche Fluoreszenzmarker eignen sich für kompetitive Assays, bei denen der Fluoreszenzmarker mit demselben, nicht fluoreszenzmarkierten biologischen Analyten um die Bindung an dem Fängermolekül konkurriert.

In einer Ausführungsform der Erfindung werden als Lumineszenzmarker unterschiedliche fluoreszenzmarkierte Antikörper verwendet, so dass man unterschiedliche Analyten bzw. Epitope in verschiedenen Fluoreszenzkanälen gleichzeitig detektieren kann (Multiplexing).

Sofern nichts anderes angegeben, wird unter der Bezeichnung "Probe" folgendes verstanden: Fluidum, Pufferlösungen aller Art, Medien für die Zellkultur und für Fermentationen, sowie Blut, Blutplasma, Urin, Serum, die alle mindestens eine Art von biologischen Analyten enthalten.

Erfindungsgemäß besonders geeignete Proben sind Fluidum, Pufferlösungen aller Art, Medien für die Zellkultur und für Fermentationen.

Sofern nichts anderes angegeben, wird unter der Bezeichnung "wässrige Lösung" folgendes verstanden: Eine wässrige, vorzugsweise pH gepufferte Lösung (z. B. ein Tris-Puffer), in der gegebenenfalls (weitere) in der Proteinbiochemie übliche Stoffe, wie z. B. Rinderalbumin (BSA), Detergenzien (z. B. Tween 20) oder Ähnliches vorhanden sind. Unter die Bezeichnung fallen auch Reaktions- und Bindepuffer. Bindepuffer bzw. Bindungspuffer sind bekannt und können den jeweiligen Versuchsanforderungen angepasst werden.

Sofern nichts anderes angegeben, wird unter der Bezeichnung "Messlösung" eine wässrige Lösung verstanden, in der die Versuchskomponenten während der Durchführung des erfindungsgemäßen Verfahrens gelöst und/oder suspendiert vorliegen.

Sofern nichts anderes angegeben, wird unter der Bezeichnung "Versuchskomponente" mindestens eine der folgenden Komponenten verstanden: Lumineszenzmarker, funktionalisierte stationäre Oberflächen, funktionalisierte Partikel und Probe sowie Fängermoleküle und primäre Antikörper.

Die Versuchskomponenten können im erfindungsgemäßen Verfahren in einem Solvent, bevorzugt in einer wässrigen Lösung, gelöst oder suspendiert verwendet werden. Gleichfalls können die Versuchskomponenten pur, d. h. ohne Zusatz eines Solvents, im erfindungsgemäßen Verfahren verwendet werden. Das ist z. B. bei einer Probe der Fall, wenn es sich um Fluidum, Pufferlösungen oder Zellkulturmedien handelt, die jeweils mindestens einen biologischen Analyten enthalten. Bevorzugte Solvents sind wässrige Lösungen wie hier definiert.

Sofern nichts anderes angegeben, wird unter einem erfindungsgemäßen biologischen Analyten oder den Begriffen "Analyt" und "Analyten" ein Molekül verstanden, das durch das erfindungsgemäße Verfahren bestimmt oder nachgewiesen werden soll. Beispiele solcher biologischer Analyten sind Proteine, Antikörper, Protein- oder Antikörperkomplexe, Peptide, DNA, RNA, Komplexe aus einem oder mehreren Biomolekülen, Viren oder eine oder mehrere biologische Zellen oder Bestandteile von diesen, sowie niedermolekulare Substanzen, die eine biologische Aktivität besitzen oder mit Biomolekülen interagieren. Im erfindungsgemäßen Verfahren sind als biologische Analyten besonders gut detektierbar: Proteine, Antikörper, Protein- oder Antikörperkomplexe, Peptide, DNA, RNA, Komplexe aus einem oder mehreren Biomolekülen sowie Viren.

Sofern nichts anderes angegeben, wird unter dem Begriff "Einbringen" folgendes verstanden: Manuelles oder automatisches Befüllen der Vorrichtung, bzw. der Messkammer durch gewöhnliche Maßnahmen wie z. B. Pipettieren oder durch Verwenden mikrofluidischer Systeme.

Sofern nichts anderes angegeben, wird unter dem Begriff "Bindung" verstanden, dass mindestens zwei Einheiten in Kontakt miteinander treten, so dass sich zwischen ihnen ein stabiles Bindungsgleichgewicht einstellt. Solche Bindungen werden im Allgemeinen durch Wechselwirkungen vermittelt und resultieren in den entsprechenden Bindungen, wie z. B. Wasserstoffbrückenbindung, Salzbindungen, Komplexbindungen und kovalente Bindungen. Die im erfindungsgemäßen Verfahren und der Vorrichtung zu eigen gemachten Bindungen sind oftmals ein Gemisch mehrerer der vorgenannten Wechselwirkungen.

Lumineszenz im Sinne der Erfindung umfasst alle bekannten Arten von Lumineszenz, insbesondere durch Licht angeregte Fluoreszenz, Bio- und Chemolumineszenz, wobei Fluoreszenz bevorzugt ist. Bei der Fluoreszenzmessung werden Moleküle mit Anregungslicht in einen angeregten elektronischen Zustand gehoben, die dann durch Abgabe der Energie, indem sie Strahlung emittieren (sog. Fluoreszenzstrahlung einer oder mehrerer bestimmter Wellenlängen oder Fluoreszenz) wieder in den energetischen Grundzustand fallen. Messung der Fluoreszenz von Molekülen ist eine etablierte Methode.

Sofern nichts anderes angegeben, wird unter dem Begriff "Magnetelement" folgendes verstanden: Beschichtung oder magnetische Schicht, wie z. B. die Folie Permaflex 518 der Fa. Rheinmagnet. Die Schichtstärke und die Folie ist so ausgewählt, dass in einer nicht-erfindungsgemäßen Ausführungsform die Magnetstärke der Folie die funktionalisierten magnetischen Partikel nach dem Vermischen nur außerhalb des Messfensters am Boden der Vorrichtung sedimentieren lässt, während das Messfenster frei bleibt, ohne jedoch das effiziente Vermischen der Versuchskomponenten (bevorzugt auf einem Schüttler) zu stören. Geeignete Feld- oder Magnetstärke sind also solche, die schwach genug sind, um ein Vermischen der Versuchskomponenten zu ermöglichen und stark genug sind, die magnetischen Partikel nach Vermischen anzuziehen, wobei das Messfenster frei von magnetischen Partikeln gehalten werden muss. Geeignete magnetische Folien sind kommerziell erhältlich. Die Messfenster, werden durch Ausstanzen oder durch Laserschneiden aus der Folie hergestellt.

Werden Protein A Mag-Sepharose® Partikel (GE Healthcare Art.-Nr. 28-9440-06) in Kombination mit der Folie Permaflex 518 der Fa. Rheinmagnet verwendet, so ist eine Schichtdicke von etwa 1 mm vorteilhaft.

Erfindungsgemäß bevorzugt werden im erfindungsgemäßen Verfahren Fluoreszenzmarker verwendet und die Messung in Schritt (d) ist eine Fluoreszenzmessung und erfolgt mit einem Fluoreszenzmessgerät.

Die erfindungsgemäße Vorrichtung und das erfindungsgemäße Verfahren erlauben es, dass eine zeitaufgelöste, kinetische Fluoreszenzmessung durchgeführt werden kann. Das ist z. B. vorteilhaft, wenn die Einstellung des Bindungs-/Reaktionsgleichgewichts verfolgt werden soll.

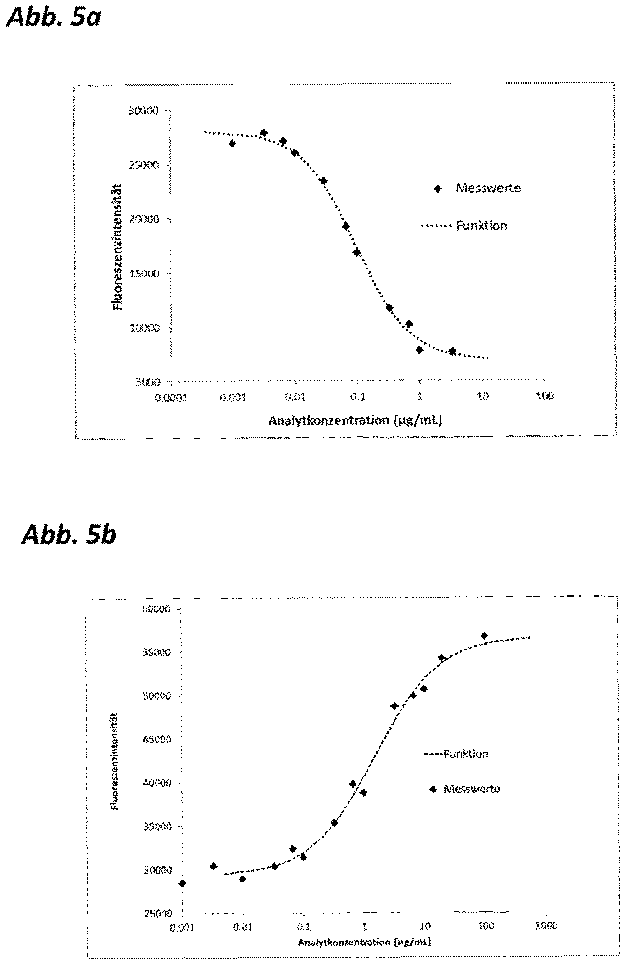

Schritt (e) im erfindungsgemäßen Verfahren, nämlich die Bestimmung der Menge und/oder Konzentration des oder der biologischen Analyten, erfolgt unter Verwendung einer Kalibrationsreihe. Für die Kalibrationsreihe wird das erfindungsgemäße Verfahren mit verschiedenen, definierten Konzentrationen/Mengen des Analyten, die den gewünschten bzw. erwarteten Konzentrationsbereich des Analyten abdecken, und jeweils identischen Versuchsbedingungen, insbesondere der Konzentration des Lumineszenzmarker, durchgeführt. Aus den gemessenen Werten der Lumineszenz-Emission des ungebundenen Lumineszenzmarkers wird dann eine Kalibrationskurve bzw. eine Funktion erzeugt, anhand derer die Analytkonzentrationen in Proben unbekannten Analytgehalts ermittelt werden. Solche Kalibrationskurven sind in den Abbildungen 5a bis 5e beispielhaft dargestellt. Vorgenannte Kalibrationsreihen und das Erstellen von Kalibrationskurven für die Ermittlung von Werten sind in der Biochemie üblich. Der Fachmann weiß wie er solche Kalibrationskurve erstellt, um aussagekräftige Ergebnisse zu erhalten.

Das erfindungsgemäße Verfahren ermittelt die Lumineszenz der in Lösung ungebunden vorliegenden Lumineszenzmarker. Das ist neu, denn die bekannten Verfahren zur Bestimmung biologischer Analyten basieren auf der Messung der an die biologischen Analyten gebundenen Lumineszenzmarker. Wird im Stand der Technik die Fluoreszenz ungebundener Lumineszenzmarker gemessen, so geht der Messung meist eine enzymatische Reaktion oder eine Spaltung, in der ein Fluoreszenzmarker von einem Detektionsantikörper abgespalten wird, voraus oder ist durch FRET oder partikuläre Sonden erzeugt. Eine solche Spaltung ist im erfindungsgemäßen Verfahren nicht vorgesehen.

Zur Bestimmung der Konzentration biologischer Analyten mittels des erfindungsgemäßen Verfahrens und wenn es sich nicht um einen kompetitiven Assay handelt, ist es vorteilhaft, wenn ein möglichst großer Anteil der biologischen Analyten an die Fängermoleküle gebunden ist. Dies wird dadurch begünstigt, dass die Fängermoleküle im Überschuss auf der Oberfläche (vorzugsweise Partikeloberfläche) vorhanden sind und eine hohe Affinität für den Analyten besitzen. Idealerweise findet die Bindungsreaktion des Analyten an die Fängermoleküle vollständig statt.

Im vorgenannten Fall hängt die Konzentration des ungebundenen Lumineszenzmarkers (z. B. Fluoreszenzmarkers) nur von der Dissoziationskonstante K_{d} zwischen Analyt und Lumineszenzmarker (z. B. Fluoreszenzmarker), der eingesetzten Konzentration des Lumineszenzmarkers (z. B. Fluoreszenzmarkers) und der zu bestimmenden Konzentration des biologischen Analyten ab.

Wird im Assay die Konzentration der Lumineszenzmarker (z. B. Fluoreszenzmarker) konstant gehalten, hängt die Konzentration der ungebundenen Lumineszenzmarker (z. B. Fluoreszenzmarkers) und somit die gemessene Lumineszenzintensität (z. B. Fluoreszenzintensität) nur von der Konzentration des biologischen Analyten ab.

Zur Bestimmung der Konzentration der biologischen Analyten mittels des erfindungsgemäßen Verfahrens und mit einem kompetitiven Assay ist es vorteilhaft, wenn die Anzahl der Fängermoleküle kleiner oder gleich der Anzahl der Lumineszenzmarker ist, damit jedes Binden eines Analytmoleküls zu einer Erhöhung der Anzahl der ungebundenen Lumineszenzmarker führt.

Zusätzlich zur Bestimmung der Konzentration des Analyten können mit dem erfindungsgemäßen Verfahren noch weitere Informationen über die Bindung des Analyten an die Versuchskomponenten erhalten werden, z. B. die Dissoziationskonstante K_{d}, oder IC- oder EC₅₀-Werte.

Durch das erfindungsgemäße Verfahren unter Verwendung der erfindungsgemäßen Vorrichtung kann die Bestimmung, insbesondere die Quantifizierung der biologischen Analyten mit einem sehr einfachen, nämlich einstufigen Assayprotokoll durchgeführt werden. Solche einstufigen Assays werden auch "mix-and-measure-assays" genannt. Im erfindungsgemäßen Verfahren werden nämlich lediglich die Versuchskomponenten zusammengegeben, und gewartet, bis sich das Bindegleichgewicht eingestellt hat.

Nach Abwarten der Sedimentation der Partikel kann sofort mit der Messung begonnen werden. Man erhält innerhalb kurzer Zeit, normalerweise in weniger als einer Stunde, die gewünschten Daten, die nach Korrelation mit der Kalibrationskurve zu dem gewünschten quantitativen oder qualitativen Ergebnis führt. Für die Messung reichen einfache, im Labor oft vorhandene Lumineszenz- bzw. Fluoreszenzdetektionsgeräte (Fluoreszenzreader).

Das erfindungsgemäße Verfahren kann als nicht-kompetitiver oder kompetitiver Assay ausgestaltet sein. Auch können mit dem erfindungsgemäßen Verfahren Sandwich-Immunoassays mit direkter und indirekter Detektion durchgeführt werden. Mit dem erfindungsgemäßen Verfahren durchzuführende bevorzugte Assays sind der direkte Assay, Sandwich Assay mit direkter oder indirekter Detektion, Verdrängungs- oder Displacement-Assay, kompetitiver Assay und sekundärer Assay.

Beim direkten Assay ist der biologische Analyt in der Regel ein Antikörper der mit einem Fängermolekül gebunden wird, der selber kein Antikörper, sondern ein Antigen, ist an das der Antikörper mit seinem variablen Teil spezifisch bindet, (schematisch dargestellt in Abbildung 6c) oder Protein A und G, welche den Antikörper an Ihrem Fc-Teil binden (schematisch dargestellt in Abbildung 6a).

In einem Inhibitionsassay, d. h. Verdrängungs- oder Displacement-Assay, verhindert der biologische Analyt ganz oder teilweise die Bindung des Lumineszenzmarkers an die funktionalisierte Oberfläche dadurch, dass der biologische Analyt an die Bindungsstelle des Lumineszenzmarkers bindet und diese nicht mehr für die Bindung an die Fängermoleküle verfügbar ist (schematisch dargestellt in Abbildung 6d).

Beim Sandwich Assay mit direkter Detektion ist der biologische Analyt in der Regel ein Protein oder ein Antikörper, das von dem variablen Teil eines Antikörper, der als Fängermolekül fungiert, gebunden wird, wobei der Lumineszenzmarker, vorzugsweise Fluoreszenzmarker, an eine zweite Bindungsstelle (Epitop) des Analyten bindet und so der Sandwich entsteht (schematisch dargestellt in Abbildung 7a).

Beim Sandwich Assay mit indirekter Detektion ist der biologische Analyt in der Regel ein Protein oder ein Antikörper, das von dem variablen Teil eines Antikörper als Fängermolekül gebunden wird, wobei Lumineszenzmarker, vorzugsweise Fluoreszenzmarker, nicht direkt an den Analyten bindet sondern an einen weiteren primären Antikörper, der an eine zweite Bindungsstelle (Epitop) des Analyten bindet (schematisch dargestellt in Abbildung 7b).

Bei allen geeigneten Assay-Varianten wird jeweils eine definierte Menge an funktionalisierten Oberflächen und Lumineszenzmarker, bevorzugt Fluoreszenzmarker, und ggf. primäre Antikörper in die erfindungsgemäße Vorrichtung bzw. Messkammer gegeben.

Beim kompetitiven Assay werden eine definierte Menge an Fängermolekülen bzw. funktionalisierten Oberflächen, die Probe und Lumineszenzmarker, bevorzugt Fluoreszenzmarker, der mit dem Analyten um die Bindung an dieselbe Bindungsstelle (Epitop) an den Fängermolekülen konkurriert, zusammen in die erfindungsgemäße Vorrichtung bzw. Messkammer gegeben. Dort wird die Reaktion unter Schütteln bis zur Einstellung des Reaktionsgleichgewichts durchgeführt und die Sedimentation der Partikel abgewartet. Anschließend wird die Menge an ungebundenem Lumineszenzmarker, bevorzugt Fluoreszenzmarker, in der Messlösung gemessen und die Konzentration des Analyten über eine Kalibrationskurve oder Eichfunktion ermittelt.

Die Konzentration des ungebundenen Lumineszenzmarker, bevorzugt Fluoreszenzmarkers, ist dabei nur abhängig von der Menge des Analyten, der die Bindung des Lumineszenzmarkers, bevorzugt des Fluoreszenzmarkers, an die Fängermoleküle ganz oder teilweise verhindert und so eine Erhöhung des Fluoreszenzsignals im Detektionsbereich bewirkt.

Die erfindungsgemäße Vorrichtung für die Verwendung im erfindungsgemäßen Verfahren ist so ausgestaltet, dass sie in der Lage ist, an funktionalisierte Oberflächen gebundene Versuchskomponenten und ungebundene Versuchskomponenten zu trennen. Hierzu enthält die Vorrichtung ein Strukturelement. Das Strukturelement ist insbesondere als Erhebung ausgestaltet, deren Querschnitt eine beliebige Geometrie aufweisen kann (z. B. kreisförmig, eckig, dreieckig), wobei das von der Basis abgewandte Ende so beschaffen ist, dass sich dort keine Versuchskomponenten ablagern wenn es sich um Partikel handelt. Das Strukturelement besitzt vorteilhafterweise eine nach oben gerade verlaufende oder sich verjüngende Erhebung auf der Basis der Vorrichtung und ist mindestens teilweise lichtdurchlässig. Das Ende kann z. B. eine konvexe Form einnehmen und/oder einen geringen Durchmesser aufweisen. Das Strukturelement ist vorzugsweise als Kegel, Kegelstumpf oder Pyramide ausgestaltet ist und ragt von der Basis der Vorrichtung hervor. Das Strukturelement kann ferner am von der Basis der Vorrichtung abgewandten Ende ein optisches Bauteil z. B. Linse aufweisen. Vorzugsweise hat es die Form eines Dorns, Kegels oder abgeschnittenen Kegels. Das Strukturelement kann aus Polypropylen bestehen und ist lichtdurchlässig. Auch andere geometrische Formen einer Erhebung, die den nachfolgend beschriebenen Zweck erfüllen, sind denkbar.

Das Strukturelement hat den Zweck, den vorgesehenen Strahlengang für die Detektion der Lumineszenz im erfindungsgemäßen Verfahren freizuhalten und zu verhindern, dass eine das Ergebnis verfälschende Lumineszenz der gebundenen Lumineszenzmarker am Messgerät ankommt. Das Vorhandensein eines Strukturelements zur Trennung ist wichtig, da die Bestimmung der biologischen Analyten über die Lumineszenz der ungebunden vorliegenden Lumineszenzmarker erfolgt. Es ist mindestens teilweise lichtdurchlässig.

Die erfindungsgemäße Vorrichtung besitzt gleichfalls einen Bereich, der als Messfenster dient. Das Messfenster kann an der Basis der Vorrichtung oder an dem der Basis gegenüber liegenden Ende der Vorrichtung vorhanden sein. Die Basis des mindestens teilweise lichtdurchlässigen Strukturelements kann ein Messfenster sein. Durch das Messfenster wird gewöhnlich angeregt und detektiert. Es ist aber auch möglich, dass das Messfenster lediglich zur Detektion benutzt wird. Die Ausgestaltung und die Lage des Messfensters innerhalb der Vorrichtung bzw. Messkammer können variieren und an die Bedürfnisse der Messung angepasst werden. Wichtig ist jedoch, dass sichergestellt ist, dass durch das Messfenster nur die Lumineszenz der ungebunden vorliegenden Lumineszenzmarker gemessen wird. Durch geeignete Auswahl der Größe des Messfensters und Art des Lumineszenzmarkers kann die Empfindlichkeit des Verfahrens, in dem die Vorrichtung benutzt wird, moduliert werden.

Der durch das Messfenster beobachtbare/spektroskopierbare Bereich innerhalb der Vorrichtung oder Messkammer, in dem die Lumineszenzmarker zur Lumineszenz emittieren, wird nachfolgend "Messbereich" oder "Detektionsbereich" genannt.

Der Bereich innerhalb der Vorrichtung oder Messkammer, der nicht vom Messfenster beobachtbar/spektroskopierbar ist, wird nachfolgend "Abtrennbereich" genannt.

In der erfindungsgemäßen Vorrichtung sind der Detektions- und Abtrennbereich verbunden, so dass der Austausch der Versuchskomponenten zwischen beiden Bereichen effektiv und leicht möglich ist. Die Konzentration des ungebundenen Lumineszenzmarker ist somit in der gesamten Vorrichtung / Messkammer gleich.

In einer bevorzugten Ausführungsform der Erfindung ist das Messfenster mit dem von der Basis abgewandten Ende des Strukturelements optisch verbunden und stellt die Grundfläche des Strukturelements dar. Beispielsweise in dem das Strukturelement eine sich nach oben gerade verlaufenden oder sich verjüngende Erhebung auf der Basis der Vorrichtung darstellt und lichtdurchlässig ist, und die Basis der Vorrichtung eine lichtundurchlässige Schicht oder Beschichtung aufweist, wobei die Basisfläche des Strukturelements ausgespart ist. Die lichtundurchlässige Schicht (z. B. eine schwarze Lackschicht) ist dabei so aufgebracht, dass sie einen Bereich auf der Basis der Vorrichtung ausspart, die unter dem Strukturelement liegt, wodurch das Messfenster gebildet wird.

Wird nun von der Basis der Vorrichtung aus, Licht in die Messkammer geschickt, dann verläuft die Strahlung durch das Strukturelement hindurch in den Messbereich hinein. Nach Anregung der Lumineszenzmarker im Messbereich, kann die Emission wieder am Messfenster gemessen werden.

In einer nicht-erfindungsgemäßen Ausführungsform des Verfahrens kann eine erfindungsgemäße Vorrichtung, Messkammer oder Mikrotiterplatte, insbesondere wie oben oder in einer der Ausführungsformen [0], [A], [B], [C], [D], [E], [F] oder [G] beschrieben, verwendet werden, die keine lichtundurchlässige Schicht aufweist, nämlich dann wenn als Fluoreszenzmessgerät ein Fluoreszenzmikroskop verwendet wird, in dem die Messoptik so angepasst ist, dass nur die Fluoreszenz-Emission aus dem Detektionsbereich detektiert wird, z. B. durch die Wahl eines Objektivs mit geeigneten Abbildungseigenschaften und einer geeigneten Sammeleffizienz (z. B. automatisiertes Fluoreszenzmikroskop des Typs NyONE (Fa. SynenTec, Elmshorn, Deutschland) inkl. 10x Objektiv der Fa. Olympus).

Werden funktionalisierte Partikel als funktionalisierte Oberflächen in der erfindungsgemäßen Vorrichtung und im erfindungsgemäßen Verfahren verwendet, so erfolgt die Trennung der ungebundenen Lumineszenzmarker von Partikel-gebundenen Lumineszenzmarkern gewöhnlich durch Sedimentation. Hierzu wird die mit Messlösung beladene Vorrichtung für eine gewisse Zeit nicht bewegt. Die funktionalisierten Partikel mit den daran gebundenen Lumineszenzmarkern besitzen eine höhere Dichte als die umgebende Messlösung und sedimentieren. Die ungebundenen Lumineszenzmarker bleiben in Lösung. Die gebundenen Lumineszenzmarker setzen sich dann um das Strukturelement auf der Basis der Vorrichtung ab, wodurch sie sich im Abtrennbereich befinden. Werden funktionalisierte Partikel im erfindungsgemäßen Verfahren verwendet, so enthält es demnach auch den Schritt (c'), nämlich Stehenlassen der Vorrichtung für eine gewisse Zeit, bevorzugt für 1 bis 30 Minuten oder 1 bis 20 bzw. 1 bis 15 Minuten, besonders bevorzugt für 1 bis 10 Minuten.

Allgemein gilt, werden nicht-erfindungsgemäße funktionalisierte magnetische Partikel verwendet, so liegt die Sedimentationsdauer der gebundenen Lumineszenzmarker bei unter 5 Minuten. Werden keine magnetischen Partikel verwendet, so liegt die Sedimentationsdauer bei unter 30 Minuten gewöhnlich im Bereich von 5 bis 20 Minuten.

Wird eine schnelle Versuchsdurchführung gewünscht, so ist es von Vorteil, funktionalisierte Partikel zu verwenden, die eine große Dichte besitzen. Bei nicht-magnetischen Partikel hat sich ein durchschnittlicher Durchmesser von etwa 90 µm als vorteilhaft erwiesen.

Große Dichte bedeutet in diesem Zusammenhang, dass die verwendeten Partikel eine größere Dichte als die wässrige Versuchslösung aufweisen.

Werden im erfindungsgemäßen Verfahren nicht-magnetische Partikel verwendet, die kleiner als 1 µm sind, dann ist es vorteilhaft, statt die Sedimentation der Partikel abzuwarten, vor der Lumineszenzmessung einen Zentrifugationsschritt einzufügen, wodurch die Partikel auf die Basis der Vorrichtung gebracht werden.

Gleichfalls ist es für eine schnelle Versuchsdurchführung vorteilhaft, wenn die funktionalisierten Partikel eine große Oberfläche besitzen, wodurch die Wahrscheinlichkeit hoch ist, dass ein biologischer Analyt bei Diffusion durch die Lösung auf ein Fängermolekül trifft. Die Wahrscheinlichkeit wird durch Schütteln (Agitation) der Vorrichtung erhöht.

Die Vorrichtung kann mit bekannten Laborgeräten und Verbrauchsmaterialien kombiniert werden, z. B. mit Mikrotiterplatten und Eppendorf-Teströhrchen, oder anderen Gefäßen und Platten, um damit das erfindungsgemäße Verfahren durchführen zu können. Die Seitenwände vorgenannter Materialien bilden dann zusammen mit der erfindungsgemäßen Vorrichtung eine Messkammer. Vorteilhafterweise werden eine Vielzahl gleichartiger Messkammern (z. B. in Form einer Mikrotiterplatte) in einem Haltersystem untergebracht, wobei es vorteilhaft ist, wenn die Abmessungen dem Standard der "Society for Biomolecular Screening" (SBS) von 127,76 mm x 85,48 mm ×14,35 mm entsprechen, um auf laborüblichen Geräten arbeiten zu können und so einen hohen Grad an Automatisierung erreichen zu können.

In einer Ausführungsform der Erfindung bezieht sich die Erfindung auf eine Messkammer, bestehend aus einer oben offenen oder geschlossenen Kammer, die Seitenwände besitzt und deren Basis durch die Vorrichtung gebildet wird. Die Messkammer kann runde oder gerade Seitenwände besitzen. Es ist aber auch möglich, dass die Messkammer mehrere Basen hat, nämlich dann wenn die Basis der Vorrichtung zusätzlich zur Basis der Messkammer vorhanden ist.

Vorzugsweise sind die Seitenwände der Messkammer und die Basis der Vorrichtung bzw. die Seitenwände der Messkammer und eine andere Basis auf der sich die Vorrichtung befindet, flüssigkeitsundurchlässig verbunden, z. B. zusammengeklebt oder geschweißt.

In einer weiteren Ausführungsform der Erfindung ist die Vorrichtung in die Vertiefung einer Mikrotiterplatte eingebracht, wobei dann die Ränder der Vertiefung die Seitenränder der Messkammer bilden.

In einer weiteren Ausführungsform der Erfindung betrifft die Erfindung eine Mikrotiterplatte in der der ursprüngliche Boden der Mikrotiterplatte gegen eine einstückige, die Strukturelemente enthaltende Basis ersetzt wurde, wobei diese Basis bis auf die Grundfläche der Strukturelemente lichtundurchlässig beschichtet ist (z. B. durch Aufkleben einer lichtundurchlässigen Folie mit entsprechenden Aussparungen oder durch Lackieren).

Die Herstellung der Vorrichtung in dem vorgenannten SBS-Format erfolgt beispielsweise indem man eine Mikrotiterplatte ohne Boden verwendet und statt des Bodens eine lichtdurchlässige (transparente) und nicht-fluoreszierende Folie (z. B. aus Polypropylen bestehend), in die die Strukturelemente in der dem SBS Format entsprechenden Anordnung eingeprägt sind, aufbringt. Solche Folien stellen eine Vielzahl von einstückigen Vorrichtungen dar. Die Strukturelemente zeigen dann in die Öffnungen der Mikrotiterplatte. Solche geprägten Folien sind käuflich erhältlich (z. B. Arraytape™ von der Firma Douglas Scientific).

Das Aufbringen der Folie auf der Seite der Mikrotiterplatte auf der eigentlich der Boden wäre, erfolgt gewöhnlich durch Verkleben mit einem Kleber, der in der Lage ist, zwei unterschiedliche Kunststoffe zu kleben. Am einfachsten wird dazu ein UV-härtender Kleber, der nicht fluoreszierend ist, benutzt. Abschließend wird eine lichtundurchlässige nichtfluoreszierende Lackschicht auf die Unterseite der Kunststofffolie in der Art aufgebracht, dass die Unterseite im Bereich der Strukturelemente ausgespart wird. Es ist auch vorstellbar, dass zuerst die Lackschicht auf die Folie aufgetragen wird und anschließend die Verklebung der Folie mit der Mikrotiterplatte erfolgt.

Die Herstellung der nicht-erfindungsgemäßen Mikrotiterplatten mit Magnetelement erfolgt durch eine gegebenenfalls reversible, d. h. fest verbundene oder lösbare Verbindung einer handelsüblichen Mikrotiterplatte mit transparentem Boden (in dem Kontext auch "Basis" genannt) mit einer magnetischen Folie einer geeigneten Magnetstärke, welche für jede Vertiefung (entspricht der Messkammer) der Mikrotiterplatte eine Aussparung aufweist, die als Messfenster genutzt wird. Die Aussparungen sind so angeordnet, dass wenn die Folie mit dem Boden der Mikrotiterplatte verbunden ist, diese sich zentriert unter den Wells der Mikrotiterplatte befinden. Die optimale Größe der Aussparung hängt von der Größe des Wells ab und kann vom Fachmann anhand des nachfolgenden Beispiels ermittelt werden. Die Durchmesser der Aussparungen müssen den Abmessungen der Wells und der Anzahl und Größe der Partikel, die im Assay verwendet werden, entsprechend dimensioniert sein. Bei einer 384-Well Mikrotiterplatte haben sich bei Verwendung von ca. 1.000 Magnetpartikeln pro Well Aussparungen mit einem Durchmesser von ca. 2,5 mm als vorteilhaft erwiesen. Dies entspricht in etwa der Hälfte der Bodenfläche einer handelsüblichen 384-well Platte und stellt einerseits genügend Fläche für das Abtrennen der magnetischen Partikel und gleichzeitig ein ausreichend großes Messfenster für die Fluoreszenzmessung zur Verfügung.

Aufgrund der besonderen Ausgestaltung der Vorrichtung bzw. der Messkammer werden die gebundenen Lumineszenzmarker bzw. Fluoreszenzmarker, die sedimentieren und hierdurch aus dem Detektionsbereich migrieren, nicht zur Lumineszenz bzw. Fluoreszenz angeregt. Die Erfindung wird anhand der nachfolgend in den Abbildungen abgebildeten und weiter unten beschriebenen Beispiele erläutert, ohne die Erfindung hierauf einzuschränken.

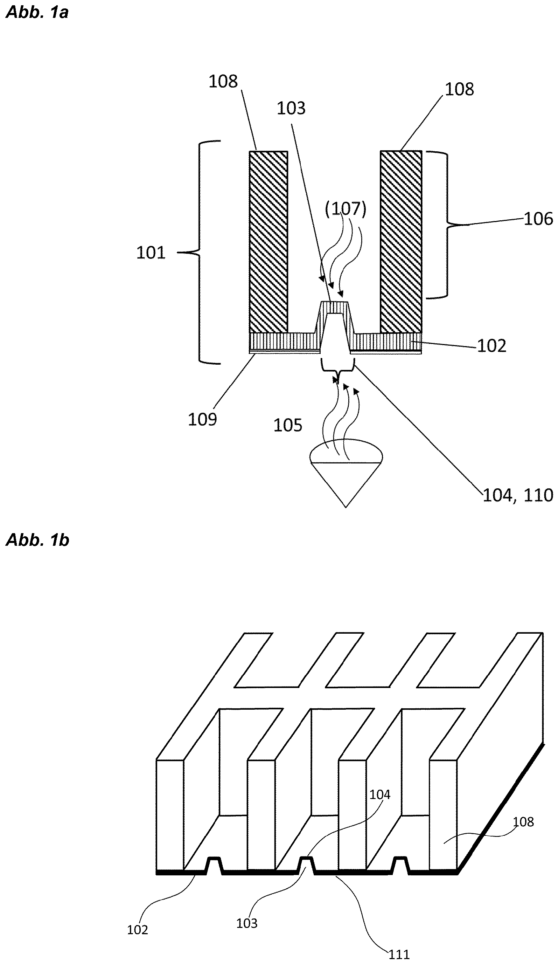

Abbildung 1a: Vertikaler Schnitt durch eine Messkammer (101) mit Strukturelement.
   Abbildung 1a zeigt eine Messkammer (101) bestehend aus einer einstückigen Vorrichtung (102) mit Strukturelement (103), das ein Messfenster (104) aufweist, durch das Anregungslicht (105) in den Detektionsbereich (106) gelangt und durch das die Emission der Lumineszenz (107) mit einem geeigneten Detektionsgerät das gleichzeitig auch das Anregungslicht liefert, oder Detektionseinheit, gemessen wird. Die einstückige Vorrichtung (102) ist flüssigkeitsundurchlässig mit den Seitenwänden (108) verbunden. Auf der unteren Seite der Basis der Vorrichtung befindet sich eine lichtundurchlässige Schicht (109) (z. B. Lack oder Folie), wobei die Grundfläche des Strukturelements (110) ausgespart ist.
Abbildung 1b: Vertikaler Schnitt durch eine Aneinanderreihung von Vorrichtungen, wie sie bspw. in eine Mikrotiterplatte integriert sein können.
   Abbildung 1b zeigt eine Vielzahl an Vorrichtungen (102), die in die Mulden/Vertiefungen einer Mikrotiterplatte eingebracht sind, wobei die Seitenwände (108) von der Mikrotiterplatte stammen und die Vorrichtung eine Folie (111) ist, in die die Strukturelemente (103) geprägt sind.

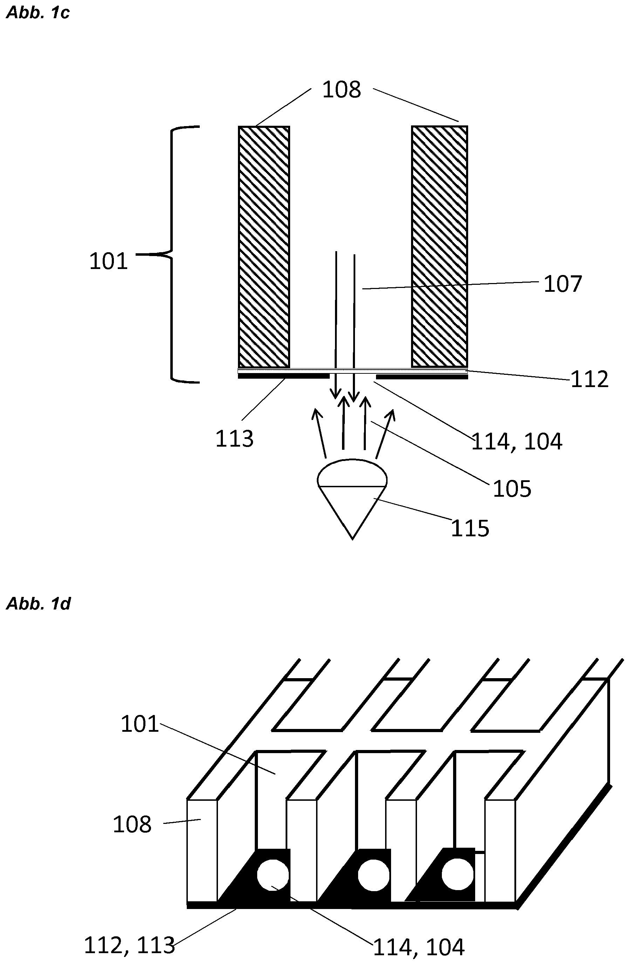

Abbildung 1c (nicht-erfindungsgemäßes Vergleichsbeispiel): Vertikaler Schnitt durch eine Messkammer (101) mit Magnetelement.
   Abbildung 1c zeigt eine nach oben offene Messkammer (101) bestehend aus einer lichtdurchlässigen Basis (112), wobei sich unterhalb der Basis ein lichtundurchlässiges magnetisches Element befindet (hier: eine lichtundurchlässige magnetische Schicht oder Folie) (113) mit Aussparung (114), wobei die Aussparung (114) das Messfenster (104) bildet. Durch das Messfenster (104) gelangt Anregungslicht (105) von unten (d. h. von der Basis der Vorrichtung aus) in die Messkammer und es kommt zu einer Emission der Lumineszenz (107) der ungebundenen Lumineszenzmarker, die dann durch das Messfenster (104) unterhalb der Vorrichtung mit Hilfe der Detektionseinheit (115) detektiert wird. Vorteilhafterweise, und wie dargestellt, kann die Detektionseinheit (z. B. ein Fluoreszenzlesegerät) Anregungslicht emittieren und die Lumineszenz detektieren. Die Basis (112) ist mit den Seitenwänden (108) und dem magnetischen Element (113) lösbar oder unlösbar verbunden. Basis und Seitenwände bestehen aus den gleichen oder unterschiedlichen Materialien.
Abbildung 1d: Vertikaler Schnitt durch eine Aneinanderreihung von Messkammern (101) (z. B. als Mikrotiterplatte ausgeführt)
   Abbildung 1d zeigt eine Vielzahl an Messkammern (101) sowie die lichtdurchlässige Basis (112) und die lichtundurchlässige magnetische Schicht oder Folie (113) mit Aussparung (114), die als Messfenster (104) dienen. Die Anregung im erfindungsgemäßen Verfahren sowie die Detektion erfolgt durch das Messfenster von unten, d. h. von unterhalb der Basis der Vielzahl der Messkammern, bzw. Mikrotiterplatte.
Abbildung 2a: Vertikaler Schnitt durch die Messkammer aus Abbildung 1a mit Messlösung nach Befüllen der Vorrichtung, jedoch vor Bindung - schematische Darstellung.

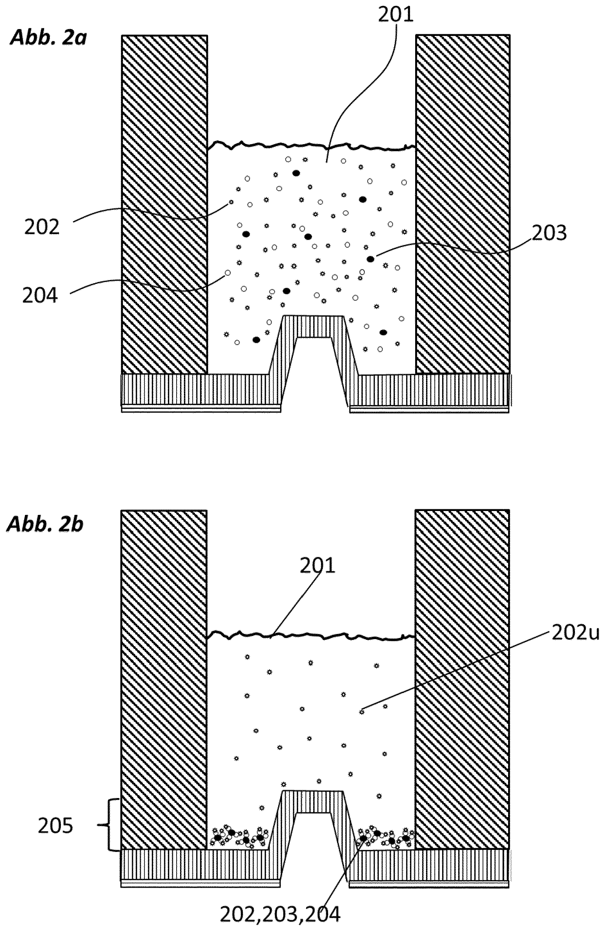

   Abbildung 2a zeigt die mit Messlösung (201) gefüllte Messkammer aus Abbildung 1a, enthaltend folgende Versuchskomponenten: biologischer Analyt (204), funktionalisierte Partikel (203), und Lumineszenzmarker (202) unmittelbar nach Einfüllen, homogen verteilt und im ungebundenen Zustand vor dem Inkontaktbringen durch Vermischen. Die funktionalisierten Partikel können auch magnetisch sein (hier nicht abgebildet).
Abbildung 2b: Vertikaler Schnitt durch die Messkammer aus Abbildung 1a mit Messlösung nach Befüllen der Vorrichtung, Bindung und Trennung - schematische Darstellung.
   Abbildung 2b zeigt die mit Messlösung (201) befüllte Messkammer aus Abbildung 2a, enthaltend die Versuchskomponenten (202), (203), (204) nach Bindung und Separation der gebundenen von den ungebundenen Lumineszenzmarkern. Die ungebundenen Lumineszenzmarker (202u) befinden sich homogen in der Messkammer verteilt, während die gebundenen Versuchskomponenten (202,203, 204) sich im Abtrennbereich (205) befinden.

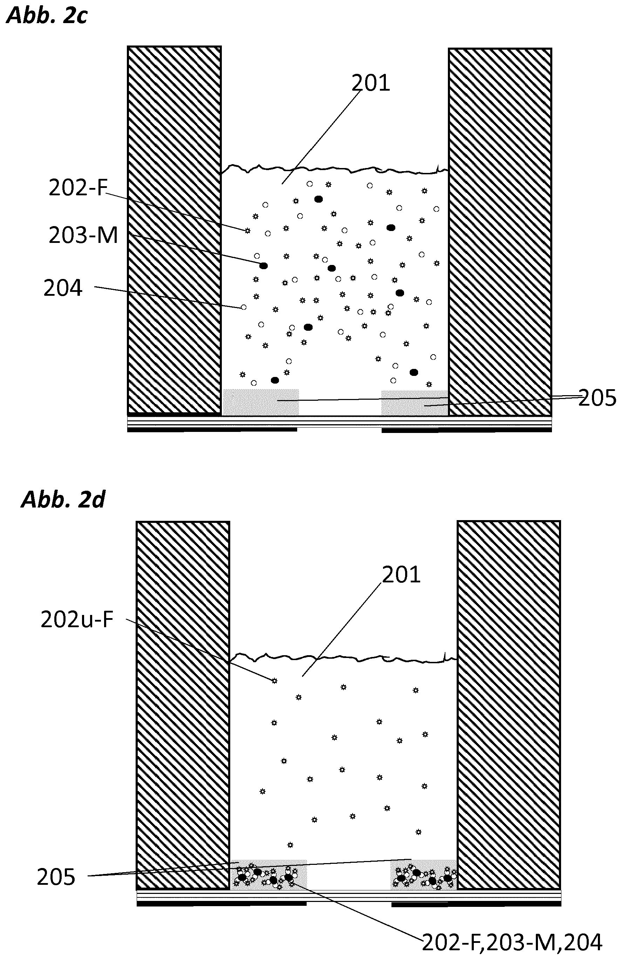

Abbildung 2c (nicht-erfindungsgemäßes Vergleichsbeispiel): Vertikaler Schnitt durch die Messkammer aus Abbildung 1c mit Messlösung nach Befüllen der Vorrichtung, jedoch vor Bindung - schematische Darstellung.
   Abbildung 2c zeigt die mit Messlösung (201) gefüllte Messkammer aus Abbildung 1c, enthaltend folgende Versuchskomponenten: biologischer Analyt (204), funktionalisierte magnetische Partikel (203-M), und Fluoreszenzmarker (202-F) unmittelbar nach Einfüllen, homogen verteilt und im ungebundenen Zustand vor dem Inkontaktbringen durch Vermischen.
Abbildung 2d: Vertikaler Schnitt durch die Messkammer aus Abbildung 1c mit Messlösung nach Befüllen der Vorrichtung, Bindung und Trennung - schematische Darstellung.
   Abbildung 2d zeigt die mit Messlösung (201) befüllte Messkammer aus Abbildung 2c, enthaltend die Versuchskomponenten (202-F), (203-M), (204) nach Bindung und Separation der gebundenen von den ungebundenen Fluoreszenzmarkern. Die ungebundenen Fluoreszenzmarker (202u-F) befinden sich homogen in der Messkammer verteilt, während die gebundenen Versuchskomponenten (202-F, 203-M, 204) sich im Abtrennbereich (205) befinden.

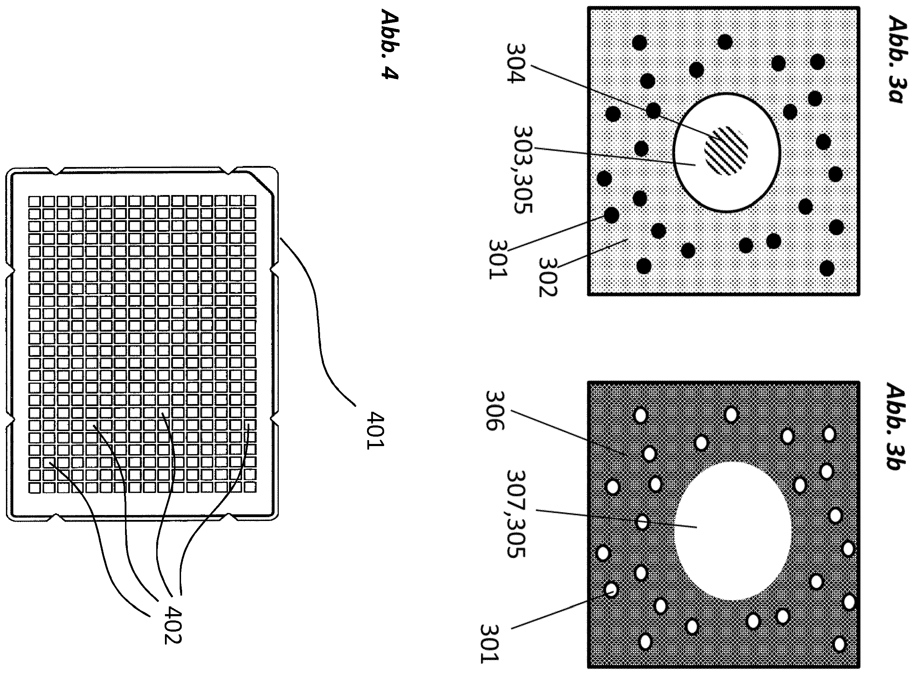

Abbildung 3a: Draufsicht in die Messkammer aus Abbildung 2b nach Befüllen der Vorrichtung, Bindung und Trennung - schematische Darstellung.
   Abbildung 3a zeigt die Messkammer aus Abbildung 2b in Draufsicht. Die gebundenen Versuchskomponenten (301) befinden sich auf der Basis der Vorrichtung/Messkammer. Die Basis der Vorrichtung ist mit einer lichtundurchlässigen Schicht (302) versehen, wobei die Grundfläche des Strukturelements (303) ausgespart ist. Die Aussparung stellt gleichzeitig das Messfenster (305) dar. Abgebildet ist auch das von der Basis der Vorrichtung abgewandte Ende (304), das mit dem Messfenster (305) optisch verbunden ist. Das Strukturelement ist hier eine sich nach oben verjüngende Erhebung mit rundem Querschnitt.
Abbildung 3b: Draufsicht in die Messkammer aus Abbildung 2d nach Befüllen der Vorrichtung, Bindung und Trennung - schematische Darstellung.
   Abbildung 3b zeigt die Messkammer aus Abbildung 2d in Draufsicht. Die gebundenen Versuchskomponenten (301) befinden sich auf der Basis der Messkammer. Die Basis der Messkammer ist mit einer lichtundurchlässigen Folie (306) mit Aussparung (307) versehen. Die Aussparung stellt gleichzeitig das Messfenster (305) dar.
Abbildung 4: Draufsicht auf eine 384 Well-Mikrotiterplatte (401) enthaltend eine Vielzahl an Messkammern (402).
Abbildung 5a: Kalibrationskurve aus Beispiel A.
Abbildung 5b: Kalibrationskurve aus Beispiel B.

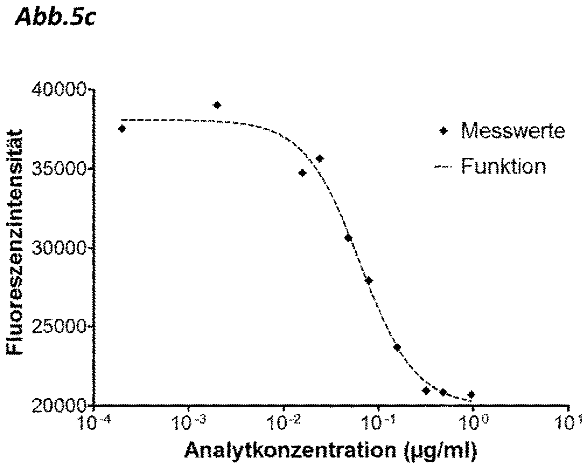

Abbildung 5c: Kalibrationsreihe aus Beispiel C.

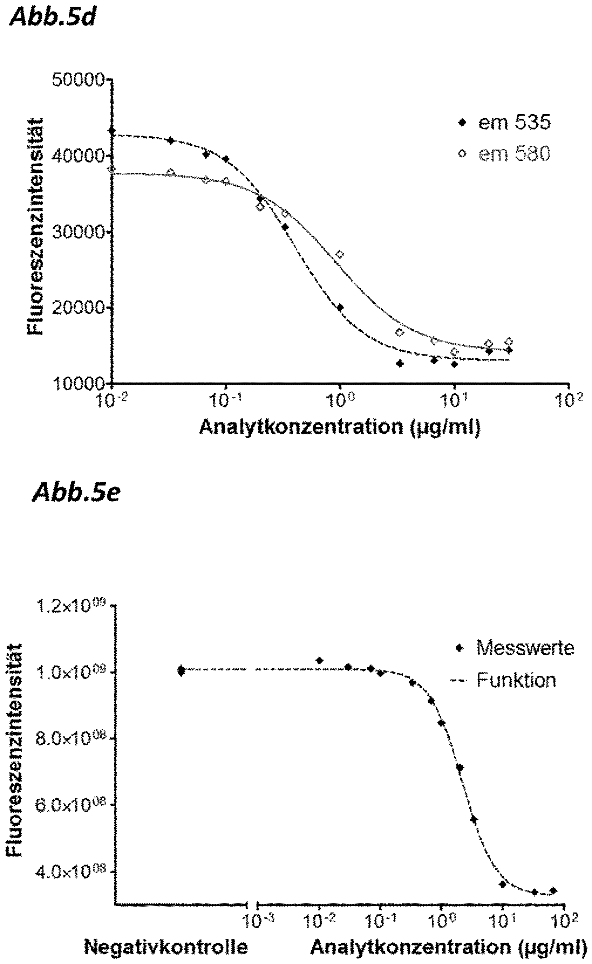

   In Abbildungen 5a, 5bund 5c ist auf der x-Achse die Analytkonzentration in µg/mL und auf der y-Achse die Fluoreszenzintensität angegeben. Durch die Messwerte (in Karo) ist eine Funktion gelegt (gestrichelte Linie).
Abbildung 5d: Messung mit zwei Fluoreszenzmarkern aus Beispiel D.
   In Abbildung 5d ist auf der x-Achse die Analytkonzentration in µg/mL und auf der y-Achse die Fluoreszenzintensität für zwei an unterschiedliche Epitope desselben Analyten bindende Fluoreszenzmarker angegeben. Die Fluoreszenzmarker emittieren auf zwei unterschiedlichen Wellenlägen (535 bzw. 580 nm). Durch die Messwerte (jeweils in Karo) sind die entsprechenden Funktionen gelegt (gestrichelte Linie).
Abbildung 5e (nicht-erfindungsgemäßes Vergleichsbeispiel): Messung eines Assays in einer Mikrotiterplatte ohne lichtdurchlässige Schicht aus Beispiel E.
   In Abbildung 5e ist auf der x-Achse die Analytkonzentration in µg/mL und auf der y-Achse die Fluoreszenzintensität angegeben. Durch die Messwerte (in Karo) ist eine Funktion gelegt (gestrichelte Linie). Da dieses Beispiel mit dem Fluoreszenzmikroskop gemessen wurde, ist die die Skalierung der y-Achse anders als in den vorhergehenden Beispielen ist.
Abbildung 6a: Schematische Darstellung des Bindeverhaltens der Versuchskomponenten in einem direkten Immunoassay für Antikörper mit einem Fängermolekül, welches den Fc-Teil des Antikörpers bindet.
   Abbildung 6a zeigt ein funktionalisiertes Partikel (601) bzw. nicht-erfindungsgemäße funktionalisiertes magnetisches Partikel (601-M) bestehend aus einem Partikel (602) bzw. (nicht-erfindungsgemäßen magnetischen Partikel (602-M) auf dem mehrere Fängermoleküle (603) vorhanden sind. Die biologischen Analyten (604) gehen mit den Fängermolekülen (603) und dem Lumineszenzmarker (607) bzw. Fluoreszenzmarker (607-F) eine Bindung ein. Weiterhin liegen ungebundene Lumineszenzmarker (607u) bzw. ungebundene Fluoreszenzmarker (607u-F) vor. Der Lumineszenzmarker bzw. Fluoreszenzmarker weist eine Bindestelle (606) und einen Lumineszenzfarbstoff (605) bzw. Fluroeszenzfarbstoff (605-F) auf.
Abbildung 6b: Schematische Darstellung des Bindeverhaltens der Versuchskomponenten in einem kompetitiven Immunoassay.
   Abbildung 6b zeigt ein funktionalisiertes Partikel (601) bzw. nicht-erfindungsgemäßes funktionalisiertes magnetisches Partikel (601-M) bestehend aus einem Partikel (602) bzw. nicht-erfindungsgemäßen magnetischen Partikel (602-M) auf dem mehrere Fängermoleküle (603) vorhanden sind. Die Fängermoleküle (603) gehen entweder mit dem biologischen Analyten (604) oder mit dem Lumineszenzmarker (607) bzw. Fluoreszenzmarker (607-F) eine Bindung, wobei der biologische Analyt (604) den gebundenen Lumineszenzmarker bzw. gebundenen Fluoreszenzmarker vom Fänger zu verdrängen vermag.
Abbildung 6c: Schematische Darstellung des Bindeverhaltens der Versuchskomponenten in einem direkten Immunoassay für Antikörper mit einem Antigen als Fängermolekül, an welchen der variable Teil des Antikörpers bindet.
   Abbildung 6c zeigt ein funktionalisiertes Partikel (601) bzw. nicht-erfindungsgemäßes funktionalisiertes magnetisches Partikel (601-M) bestehend aus einem Partikel (602) bzw. nicht-erfindungsgemäßen magnetischen Partikel (602-M) auf dem mehrere Fängermoleküle (603) vorhanden sind. Die biologischen Analyten (604) gehen mit den Fängermolekülen (603) und dem Lumineszenzmarker (607) bzw. Fluoreszenzmarker (607-F) eine Bindung ein. Weiterhin liegen ungebundene Lumineszenzmarker (607u) bzw. ungebundene Fluoreszenzmarker (607u-F) vor. Der Lumineszenzmarker weist eine Bindestelle (606) und einen Lumineszenzfarbstoff (605) bzw. Fluoreszenzfarbstoff (605-F) auf.
Abbildung 6d: Schematische Darstellung des Bindeverhaltens der Versuchskomponenten in einem Inhibitionsassay fürAntikörper.
   Abbildung 6d zeigt ein funktionalisiertes Partikel (601) bzw. nicht-erfindungsgemäßes funktionalisiertes magnetisches Partikel (601-M) bestehend aus einem Partikel (602) bzw. nicht-erfindungsgemäßen magnetischen Partikel (602-M) auf dem mehrere Fängermoleküle (603) vorhanden sind, wobei die Fängermoleküle (603) den Fluoreszenzmarker (607-F), bestehend aus Bindestelle (606) und Fluoreszenzfarbstoff (605-F) binden können. Der biologische Analyt (604) bindet an den Fluoreszenzmarker (607-F), und verhindert (inhibiert) dadurch die Bindung des Fluoreszenzmarkers an den Fänger (603). Bei dieser Art von Bindung wird der Komplex (608, 608-F), detektiert nämlich der an den biologischen Analyten (604) gebundene Lumineszenzmarker (607) bzw. Fluoreszenzmarker (607-F).
Abbildungen 7a und 7b: Schematische Darstellung des Bindeverhaltens der Versuchskomponenten in einem Sandwich-Immunoassay mit direkter Detektion (Abb. 7a) und indirekter Detektion (Abb. 7b).
   In beiden Abbildungen ist jeweils ein funktionalisiertes Partikel (701) bzw. nicht-erfindungsgemäßes funktionalisiertes magnetisches Partikel (701-M) gezeigt, das aus einem Partikel (702) bzw. nicht-erfindungsgemäßen magnetischen Partikel (702-M) besteht, auf dem mehrere Fängermoleküle (703) gebunden sind. Das Fängermolekül wird aus einem geeigneten Protein (705) z. B. biotinylierter Antikörper, das über einen Linker (704) (z. B. Streptavidin) an den Partikel (702) bzw. magnetischen Partikel (702-M) gebunden wird, gebildet. Das Protein (705) bindet den biologischen Analyten (709). Abgebildet sind weiterhin gebundene Lumineszenzmarker (706) bzw. gebundene Fluoreszenzmarker (706-F) und ungebundene Lumineszenzmarker (706u) bzw. ungebundene Fluoreszenzmarker (706u-F). Der Lumineszenzmarker (706) bzw. Fluoreszenzmarker (706-F) weist eine Bindestelle (707) und einen Lumineszenzfarbstoff (708) bzw. Fluoreszenzfarbstoff (708-F) auf.
In Abbildung 7b ist neben den in Abbildung 7a dargestellten Versuchskomponenten zusätzlich noch ein primärer Antikörper (710) vorhanden, der an den Analyten (709) bindet, und an den der Lumineszenzmarker (706) bzw. Fluoreszenzmarker (706-F) bindet.

Das erfindungsgemäße Verfahren kann unter Verwendung der erfindungsgemäßen Messkammer, wie in Abbildung 1a dargestellt, wie folgt durchgeführt werden:
In die Messkammer (101) wird eine Messlösung (201), welche biologische Analyten (204), funktionalisierte Partikel (203) und ungebundene Lumineszenzmarker (202) enthält, eingebracht (siehe Abbildung 2a). Es entsteht eine homogene Mischung. Nach Ausbildung der entsprechenden Bindungen (durch Inkubation und Schütteln) sedimentieren die gebundenen Versuchskomponenten in den Abtrennbereich (205), und sammeln sich dort (siehe Abbildungen 2b und 3a). Im Detektionsbereich (106) können nun die ungebundenen Lumineszenzmarker (202u) vermessen werden. Die lichtundurchlässige Schicht (109) sorgt dafür, dass die sich im Abtrennbereich befindenden gebundenen Lumineszenzmarker weder durch Anregungslicht zur Emission angeregt werden können, noch dass das Emissionslicht der gebundenen Lumineszenzmarker durch das Messfenster aus der Messkammer zur Detektionseinheit /-gerät gelangen.

Durchführung eines nicht-erfindungsgemäßes Vergleichsbeispiel unter Verwendung der nicht-erfindungsgemäßen Messkammer mit Magnetelementen, wie in Abbildung 1c dargestellt kann wie folgt erfolgen:
In die Messkammer (101) wird eine Messlösung (201), welche biologische Analyten (204), funktionalisierte magnetische Partikel (203-M) und ungebundene Fluoreszenzmarker (202-F) enthält, eingebracht (siehe Abbildung 2c). Es entsteht eine homogene Mischung. Nach Ausbildung der entsprechenden Bindungen (durch Inkubation und Schütteln) sedimentieren die gebundenen Versuchskomponenten durch das Magnetfeld des Magnetelements gerichtet in den Abtrennbereich (205), und sammeln sich dort (siehe Abbildungen 2d und 3b). Im Detektionsbereich können nun die ungebundenen Fluoreszenzmarker (202u-F) vermessen werden. Die lichtundurchlässige magnetische Schicht oder Folie (113) sorgt dafür, dass die sich im Abtrennbereich befindenden gebundenen Fluoreszenzmarker (202-F) weder durch das Anregungslicht zur Emission angeregt werden können, noch dass das Emissionslicht der gebundenen Fluoreszenzmarker (202-F) durch das Messfenster (104) aus der Messkammer zur Detektionsvorrichtung (115) gelangen.

Beispielhafte Durchführungen von Kalibrierungen für das erfindungsgemäße Verfahren werden nachstehend beschrieben. Die Bestimmung des in der Kalibrierungsreihe verwendeten biologischen Analyten erfolgt in analoger Weise zu nachfolgenden Beispielen an Proben unbekannten Analytgehalts, wobei die Analytkonzentration über die gemessenen Fluoreszenzintensität der ungebundenen Fluoreszenzmarker unter Verwendung der jeweiligen Kalibrationskurve ermittelt wird.

### Beispiel A: Messung einer Kalibrierungsreihe in der direkten Assayvariante

Für die Messung wird folgende erfindungsgemäße Mikrotiterplatte verwendet: Eine schwarze Mikrotiterplatte (Fa. Greiner BioONE, Art.-Nr. 781000-06) mit 384 Wells (Messkammern), in denen sich jeweils ein 1,6 mm hohes, aus Polypropylen bestehendes konisches Strukturelement, das sich nach oben verjüngt und einen runden Querschnitt aufweist, befindet, das an der der Basis abgewandten Seite einen Durchmesser von 1 mm aufweist. Die Messkammern sind an ihrer Unterseite mit einer lichtundurchlässigen Lackschicht versehen, wobei die Schicht nicht auf der Basisfläche des Strukturelements aufgetragen wird, so dass an der Basis ein Messfenster entsteht, das einen Durchmesser von ca. 2 mm hat.

Als funktionalisierte Partikel werden Protein A-Sepharose® 4B, Fast Flow Beads (Sigma-Aldrich, Art.-Nr. P9424) mit einem durchschnittlichen Durchmesser von 90 µm verwendet.

Als Fluoreszenzmarker wird ein mit Alexa 647-konjugiertes Antikörperfragment verwendet (Jackson Immuno Research, AffiniPure F(ab')₂ Fragment Goat Anti-Human IgG F(ab')₂ specific Art.-Nr. 109-606-097).

Als wässrige Lösung wird folgender Puffer verwendet: 10 mM Tris, 150 mM NaCl, 0,1% Rinderalbumin (BSA), 0,05% Polysorbat 20 (Tween 20™), pH 7,4 in destilliertem Wasser (hiernach Puffer genannt). Als biologischer Analyt für die Kalibration wird der rekombinant hergestellte Antikörper Rituximab (Mabthera™) verwendet.

In der Mikrotiterplatte wird eine Kalibrationskurve mit insgesamt 12 verschiedenen Analytkonzentrationen erstellt. Die Analytkonzentrationen betragen 0/0,001/0,0033/0,0067/ 0,01/0,03/0,67/0,1/0,33/0,67/1,0 und 3,33 µg/mL Rituximab (Mabthera™).

Hierzu wird wie folgt vorgegangen: In jede Messkammer werden 54 µL einer Stammlösung, die 15 µL Protein A Sepharose Beads Slurry und 60 µL Fluoreszenzmarker (10 µg/mL) in 810 µL Puffer enthält, gegeben.

Aus einer konzentrierten Stammlösung des Analyten (10 mg/mL) werden Verdünnungen von 1:300 bis 1:1.000.000 in Puffer erstellt und davon [Aliquote von] jeweils 6 µL in die Messkammer gegeben, um die Zielkonzentrationen an Analyten für die Kalibrationsreihe zu erreichen.

Die gesamte Mikrotiterplatte wird bei Raumtemperatur bei 1400 rpm auf einem Eppendorf Thermomixer Comfort 45 Minuten lang geschüttelt. Danach wird die Mikrotiterplatte vom Thermoschüttler entfernt und 5 Minuten gewartet, bis die Partikel sedimentiert sind.

Anschließend erfolgt die Messung der Fluoreszenzintensität von unten (bottom reading) in jeder Messkammer in einem Tecan InfiniteM100 Fluoreszenzplattenreader bei einer Anregungswellenlänge von 645 nm und einer Emissionswellenlänge von 675 nm.

Die erhaltenen Werte für die Fluoreszenzintensität werden gegen die Analytkonzentrationen aufgetragen und mit einem 4-Parameterfit gefittet um die Kalibrationsfunktion zu erhalten. Die entsprechende Kalibrationskurve ist in Abbildung 5a dargestellt.

### Beispiel B: Messung einer Kalibrierungsreihe im kompetitiven Assay

Für die Messung wird folgende erfindungsgemäße Mikrotiterplatte verwendet: Eine schwarze Mikrotiterplatte (Fa. Greiner BioONE, Art.-Nr. 781000-06) mit 384 Wells (Messkammern), in denen sich jeweils ein 1,6 mm hohes aus Polypropylen bestehendes konisches Strukturelement, das sich nach oben verjüngt und einen runden Querschnitt aufweist, befindet, das an der der Basis abgewandten Seite einen Durchmesser von 1 mm aufweist. Die Messkammern sind an Ihrer Unterseite mit einer lichtundurchlässigen Lackschicht versehen, wobei die Schicht nicht auf der Basisfläche des Strukturelements aufgetragen wird, so dass an der Basis ein Messfenster entsteht, das einen Durchmesser von ca. 2 mm hat.

Als funktionalisierte Partikel werden Streptavidin Mag-Sepharose® Partikel (VWR, Art.-Nr. 28-9857-38) mit einem durchschnittlichen Durchmesser von 70 µm verwendet, die mit einem biotinylierten Protein A-Fragment (Fa. Affibody, Art. No. 10.0623.02.00005) funktionalisiert sind. Die Funktionalisierung der Partikel erfolgt durch eine halbstündige Inkubation von 100 µL Partikelsuspension mit 0,3 µL einer Lösung des biotinylierten Protein A-Fragments mit einer Konzentration von 1 mg/mL und anschließendem Waschen der Partikel mit Puffer.

Als Fluoreszenzmarker wird ein mit Alexa 488-konjugierter polyklonaler Kaninchen anti-Huhn IgY (H+L)-Alexa Fluor 488 verwendet (Jackson Immuno Research, Art.-No. 303-545-003).

Als wässrige Lösung wird folgender Puffer verwendet: 10 mM Tris, 150 mM NaCl, 0,1% Rinderalbumin (BSA), 0,05% Polysorbat 20 (Tween™ 20), pH 7,4 in destilliertem Wasser (hiernach Puffer genannt). Als biologischer Analyt für die Kalibrationsreihe wird der rekombinant hergestellte Antikörper Rituximab (Mabthera™) verwendet.

In der Mikrotiterplatte wird eine Kalibrationskurve mit insgesamt 14 verschiedenen Analytkonzentrationen erstellt. Die Analytkonzentrationen betragen 0,001/0,0033//0,01/0,03/ 0,67/0,1/0,33/0,67/1,0/3,33/6,67/10/20 und 100 µg/mL Rituximab (Mabthera™).

Hierzu wird wie folgt vorgegangen: Beladen der Messkammer mit funktionalisierten Partikel in dem in jede Messkammer 48 µL einer Stammlösung enthaltend 80 µL einer Suspension vorgenannter funktionalisierter Partikel in 1840 µL Puffer, gegeben wird.

Erstellen von Mischungen aus Fluoreszenzmarker in jeweils gleichen Konzentrationen und biologischen Analyten in unterschiedlichen Konzentrationen, in dem aus einer konzentrierten Stammlösung des Analyten (10 mg/mL) Verdünnungen von 1:10 bis 1:1.000.000 in Puffer erstellt werden. Von der Verdünnungen werden Aliquote von jeweils 6 µL mit jeweils 6 µL des Kaninchen anti-Huhn IgY (H+L)-Alexa Fluor 488 Antikörpers (aus einer Stocklösung von 10 µg/mL) zusammengeben und gemischt.

Einfüllen der Mischung aus Fluoreszenzmarker und biologischen Analyten in die Messkammer. Vermischen der Versuchskomponenten, indem die Mikrotiterplatte bei Raumtemperatur bei 1600 rpm auf einem Eppendorf MixMate 30 Minuten lang geschüttelt wird. Nach Beendigung des Schüttelns wird die Mikrotiterplatte vom Schüttler genommen und solange gewartet, bis die Partikel sedimentieren, was hier ca. 1-2 Minuten dauerte.

Überführen der Platte in ein Fluoreszenzaufnahmegerät, das für Messungen von unten (sog. bottom-reading) geeignet ist (z. B. Tecan Safire Monochromatic Fluorescence reader), in der jede Messkammer von unten bei einer gewissen Anregungswellenlänge (hier 488 nm) beleuchtet wird und die resultierende Fluoreszenz-Emission bzw. Fluoreszenzintensität bei einer gewissen Emissionswellenlänge (hier 535 nm) aufgenommen wird.

Auftragen der erhaltenen Werte für die Fluoreszenzintensität gegen die Analytkonzentrationen und fitten mit einem 4-Parameterfit, um die Kalibrationsfunktion zu erhalten. Die entsprechende Kalibrationskurve ist in Abbildung 5b dargestellt.

### Beispiel C: Messung einer Kalibrierungsreihe in Mikrotiterplatte mit Magnetelement

Für die Messung wird folgende erfindungsgemäße Mikrotiterplatte verwendet: Eine schwarze Mikrotiterplatte mit transparentem Boden (Fa. Greiner BioONE, Art.-Nr. 781091) mit 384 Wells, unter die eine Magnetfolie mit 384 Aussparungen geklebt wird, so dass jede Aussparung sich zentriert unter den Wells der Mikrotiterplatte befindet. Die dauermagnetische Folie (Permaflex 518, Fa. Rheinmagnet) hat eine Dicke von 1 mm und die Aussparungen einen Durchmesser von 2,5 mm.

Als funktionalisierte magnetische Partikel werden Protein A Mag-Sepharose® Partikel (GE Healthcare Art.-Nr. 28-9440-06) mit einem Durchmesser von 37-100 µm verwendet.

Als Fluoreszenzmarker wird ein mit Fluorescin-Isothiocyanat (FITC)-konjugierter polyklonaler Huhn anti-human IgG (H+L) Antikörper verwendet (Fa. Abcam, Art.-Nr. 112453).

Als wässrige Lösung wird folgender Puffer verwendet: 10 mM Tris, 150 mM NaCl, 0,1% Rinderalbumin (BSA), 0,05% Polysorbat 20 (Tween™ 20), pH 7,4 in destilliertem Wasser. Als biologischer Analyt wird der Antikörper Rituximab (Mabthera™) verwendet.

Der Assay wurde wie in Beispiel A beschrieben durchgeführt, wobei folgende Partikel und Fluoreszenzmarker enthaltende Stammlösung verwendet wird: 75 µL einer Suspension von Protein A Mag-Sepharose® Partikel und 1,86 µL des Fluoreszenzmarkers in 1782 µL Puffer.

Davon wurden jeweils 47 µL mit je 3 µL einer Probe aus der Kalibrationsreihe von Rituximab für 30 Minuten auf einem Schüttler Variomag Monoshake (Firma H+P) für 30 Minuten inkubiert. Anschließend wurde die Mikrotiterplatte 5 Minuten stehengelassen, um die Sedimentation der Partikel abzuwarten, und anschließend gemessen.

Die Messung erfolgt in einem Tecan Safire Fluorescencereader bei einer Anregungswellenlänge von 490 nm und einer Emissionswellenlänge von 535 nm.

Die Kalibrationsfunktion wird, wie in Beispiel A beschrieben, ermittelt und die entsprechende Kalibrationskurve ist in Abbildung 5c dargestellt.

### Beispiel D: Messung mit zwei Fluoreszenzmarkern

Für die Messung wird folgende erfindungsgemäße Mikrotiterplatte verwendet: Eine schwarze Mikrotiterplatte (Fa. Greiner BioONE, Art.-Nr. 781000-06) mit 384 Wells (Messkammern) und einem einstückigen, Strukturelemente enthaltenen Boden, der durch Thermoformen hergestellt wurde. Die Strukturelemente weisen die Form einer vierseitigen Pyramide auf einem quadratischen Sockel auf. Die Messkammern sind an ihrer Unterseite mit einer lichtundurchlässigen Lackschicht versehen, wobei die Schicht nicht auf der quadratischen Basisfläche des Strukturelements aufgetragen wird, so dass an der Basis ein Messfenster entsteht, das einen Durchmesser von ca. 2,5 mm hat.

Dieser Assay wird unter Verwendung von Partikeln durchgeführt, die bereits getrocknet in den Mikrotiterplatten vorgelegt wurden. Dazu werden jeweils 30 µL einer Suspension von Protein A-Sepharose® 4B Fast Flow Beads auf der Mikrotiterplatte bei 35 Grad Celsius eingetrocknet. Eine Stammlösung aus 1,6 µL eines ersten Fluoreszenzmarkers, nämlich Alexa 488 AffiniPure F(ab')₂ Fragment Goat Anti-Human IgG, F(ab')₂ specific (Jackson Immuno Research Art.-Nr. 109-546-097), und 3,93 µL eines zweiten Fluoreszenzmarkers, nämlich R-Phycoerythrin AffiniPure F(ab')₂ Fragment GoatAnti-Human IgG, Fcγ Fragment specific (Jackson Immuno Research Art.-Nr. 109-116-170), in 1607 µL Puffer werden angesetzt und davon jeweils 54 µL mit je 6 µL einer Probe aus der Kalibrationsreihe von Rituximab (Mabthera™), für 30 Minuten auf einem Schüttler Variomag Monoshake (Firma H+P) inkubiert. Anschließend wurde die Mikrotiterplatte 15 Minuten stehengelassen um die Sedimentation der Partikel abzuwarten und anschließend gemessen.

Als wässrige Lösung wird folgender Puffer verwendet: 10 mM Tris, 150 mM NaCl, 0,1% Rinderalbumin (BSA), 0,05% Polysorbat 20 (Tween™ 20), pH 7,4 in destilliertem Wasser.

Die Messung erfolgt in einem Tecan Safire Fluorescencereader bei einer Anregungswellenlänge von 490 nm und bei Emissionswellenlängen von 535 nm zur Detektion des ersten Fluoreszenzmarker (=Alexa 647-konjugiertes Antikörperfragment verwendet (Jackson Immuno Research, AffiniPure F(ab')₂ Fragment Goat Anti-Human IgG F(ab') specific 488) und 580 nm für die Detektion des zweiten (=R-Phycoerythrin).

Analog den Beispielen A und B werden die jeweiligen Kalibrationsfunktionen ermittelt. Die entsprechende Kalibrationskurve ist in Abbildung 5d dargestellt. Abbildung 5d zeigt ferner, dass die gleichzeitige Detektion verschiedener Bindungsstellen am Analyten (hier: Rituximab (Mabthera™)) mit ähnlichen Kalibrationskurven möglich ist.

### Beispiel E: Messung eines Assays in einer Mikrotiterplatte ohne lichtundurchlässige Schicht

Für die Messung wird die im Beispiel D beschriebene Mikrotiterplatte mit Strukturelementen verwendet, allerdings ohne die lichtundurchlässige Lackschicht.

Als wässrige Lösung wird folgender Puffer verwendet: 10 mM Tris, 150mM NaCl, 0,1% Rinderalbumin (BSA), 0,05 % Polysorbat 20 (Tween™ 20), pH 7,4 in destilliertem Wasser.

Die Durchführung des Assays entspricht Beispiel A unter Verwendung folgender Stammlösung mit funktionalisierten Partikeln und Fluoreszenzmarker: 600 µL einer vorverdünnten Suspension Protein A-Sepharose® 4B Fast Flow Beads und 1,06 µL des Fluoreszenzmarkers Alexa488 AffiniPure F(ab')₂ Fragment Goat Anti-Human IgG, F(ab')₂ specific (Jackson Immuno Research Art.-Nr. 109-546-097) in 478 µL Puffer. Davon werden jeweils 54 µL mit je 6 µL einer Probe aus der Kalibrationsreihe von Rituximab (Mabthera™) wie in den Beispielen A beschrieben für 30 Minuten auf einem Schüttler Variomag Monoshake (Firma H+P) für 30 Minuten inkubiert. Anschließend wird die Mikrotiterplatte 15 Minuten stehengelassen, um die Sedimentation der Partikel abzuwarten und anschließend gemessen.

Für die Messung wird ein automatisiertes Fluoreszenzmikroskop des Typs NyONE (Fa. SynenTec, Elmshorn, Deutschland) eingesetzt. Mit einem 10x Objektiv der Fa. Olympus wird jeweils ein zentral in der Mitte des Wells positioniertes Bild aufgenommen und die Fluoreszenzintensität im Bild gemessen.

Analog des Beispiels A wird eine Kalibrationsfunktion ermittelt. Die entsprechende Kalibrationskurve ist in Abbildung 5e dargestellt.

## Patentansprüche

1. Verfahren zur quantitativen Bestimmung von biologischen Analyten in einer wässrigen Lösung in Gegenwart einer oder mehrerer funktionalisierter Oberflächen, wobei die wässrige Lösung mindestens eine Art von biologischen Analyten und mindestens eine Art Fluoreszenzmarker enthält, **dadurch gekennzeichnet, dass** zur Bestimmung der Menge und/oder Konzentration des oder der biologischen Analyten die Fluoreszenz-Emission der ungebundenen Fluoreszenzmarker gemessen wird, und in dem als funktionalisierte Oberflächen funktionalisierte Partikel aus Polymer oder einem Polymergemisch verwendet werden, die mittlere Durchmesser im Bereich von 20 µm bis 200 µm aufweisen und auf deren Oberfläche Fängermoleküle vorhanden sind, die mit dem oder den biologischen Analyten und/oder mit dem oder den Fluoreszenzmarkern eine Bindung eingehen, unter Verwendung einer Messkammer (101), bestehend aus einer oben offenen oder geschlossenen Kammer, die Seitenwände besitzt und deren Basis durch eine Basis einer Vorrichtung gebildet wird, wobei die Vorrichtung ein mindestens teilweise lichtdurchlässiges Strukturelement (103), aufweist das als eine sich nach oben verjüngende Erhebung ausgestaltet ist, deren Querschnitt eine beliebige Geometrie aufweist, wobei das von der Basis der Vorrichtung abgewandte Ende des Strukturelements (304) flach oder konvex geformt ist und so beschaffen ist, dass sich dort keine Partikel ablagern und der Boden der Messkammer bis auf einen unter dem Strukturelement liegenden Bereich (110) lichtundurchlässig ist.

2. Das Verfahren nach Anspruch 1, **dadurch gekennzeichnet, dass** das Strukturelement die Form eines Dorns, Kegels, abgeschnittenen Kegels, Pyramide, abgeschnittener Pyramide deren Grundfläche n-eckig ist, wobei n für eine Zahl im Bereich von 3 bis 10 steht, aufweist und lichtdurchlässig ist.

3. Das Verfahren nach Anspruch 1 oder 2, umfassend die Schritte:
(a) Einbringen mindestens einer Art funktionalisierter Partikel in die Messkammer
(b) Einbringen einer Probe enthaltend mindestens eine Art biologischer Analyt in die Messkammer;
(c) Einbringen mindestens einer Art Fluoreszenzmarker in die Messkammer;
(c') Vermischen der funktionalisierten Partikel, Probe und Fluoreszenzmarker in der Messkammer;
(c") Trennung der ungebundenen von den gebundenen Fluoreszenzmarkern, so dass die gebundenen Fluoreszenzmarker sich im Abtrennbereich (205) befinden;
(d) Messen der Fluoreszenz-Emission der ungebundenen Fluoreszenzmarker im Detektionsbereich (106); und
(e) Bestimmen der Menge und/oder Konzentration des oder der biologischen Analyten.

4. Das Verfahren nach Anspruch 3, **dadurch gekennzeichnet, dass** die Schritte (a), (b) und (c) sequentiell erfolgen oder mindestens zwei der Schritte erfolgen gleichzeitig.

5. Das Verfahren nach Anspruch 3 **dadurch gekennzeichnet, dass** Schritt (a) und/oder Schritt (c) vor und in einem zeitlichen Abstand zu den übrigen Schritten stattfinden, wobei der zeitliche Abstand größer als 12 Stunden ist.

6. Das Verfahren nach einem der Ansprüche 1 bis 5, wobei es sich bei dem Verfahren um einen direkten Immunoassay, Sandwich Immunoassay, Verdrängungs- oder Displacement-Immunoassay, kompetitiven Immunoassay und sekundären Immuno-assay handelt.

7. Mikrotiterplatte zur Durchführung des Verfahrens gemäß einem der Ansprüche 1 bis 6, in der mindestens ein Well als eine Meßkammer (101) ausgestaltet ist, die Messkammer bestehend aus einer oben offenen oder geschlossenen Kammer, die Seitenwände besitzt und deren Basis durch die Basis einer Vorrichtung gebildet wird, wobei die Vorrichtung ein mindestens teilweise lichtdurchlässiges Strukturelement (103), aufweist das als eine sich nach oben verjüngende Erhebung ausgestaltet ist, deren Querschnitt eine beliebige Geometrie aufweist, wobei das von der Basis der Vorrichtung abgewandte Ende des Strukturelements (304) flach oder konvex geformt ist und so beschaffen ist, dass sich dort keine Partikel ablagern und der Boden der Messkammer bis auf einen unter dem Strukturelement liegenden Bereich (110) lichtundurchlässig ist.

8. Die Mikrotiterplatte nach Anspruch 7, **dadurch gekennzeichnet, dass** sich in mindestens einer der Messkammern funktionalisierte Partikel und/oder Fluoreszenzmarker in getrockneter Form befinden.

9. Ein Kit zur Durchführung des Verfahrens nach einem der Ansprüche 1 bis 6 enthaltend eine Mikrotiterplatte gemäß Anspruch 7, mindestens eine Art funktionalisierter Partikel, mindestens eine Art Fluoreszenzmarker und einen Reaktionspuffer.

10. Verfahren zur Herstellung einer Mikrotiterplatte nach Anspruch 7, umfassend die folgenden Schritte:
(x) Ersetzen des Bodens einer Mikrotiterplatte durch eine Strukturelemente aufweisende Basis, die vorzugsweise so viele Strukturelemente aufweist, wie in der Mikrotiterplatte Wells vorhanden sind;
(y) Verbinden der Basis mit der Mikrotiterplatte; und
(z) Aufbringen einer lichtundurchlässigen Schicht auf die Unterseite der Basis, wobei der unter dem Strukturelement liegende Bereich, nämlich die Grundfläche des Strukturelements (110), ausgespart wird.

## Claims

1. A method for the quantitative determination of biological analytes in an aqueous solution in the presence of one or more functionalised surfaces, wherein the aqueous solution contains at least one type of biological analytes and at least one type of fluorescence marker, **characterised in that** the fluorescence emissions of the unbound fluorescence markers are measured in order to determine the quantity and/or concentration of the biological analyte or analytes and in which functionalised particles made from polymer or a polymer mixture which have an average diameter in a range of between 20 µm and 200 µm and which have capture molecules on their surface which bond with the biological analyte or analytes and/or with the fluorescence marker or markers are used as functionalised surfaces, using a measuring chamber (101) comprising a topless or closed chamber which has side walls and the base of which is formed by a base of a device, wherein the device has an at least partially translucent structural element (103) which is designed as a protrusion which tapers upward, the cross section of which has any geometry, wherein the end of the structural element (304) which is facing away from the base of the device has a flat or convex shape and is provided such that no particles can settle there and the base of the measuring chamber, apart from the base area of the structural element (110), is opaque.

2. The method according to claim 1, **characterised in that** the structural element has the form of a mandrel, cone, truncated cone, pyramid, truncated pyramid with an n-cornered base, wherein n is a number in the range between 3 and 10, and is translucent.

3. The method according to claim 1 or 2, including the steps:
(a) Introduction of at least one type of functionalised particle into the measuring chamber;
(b) Introduction of a sample containing at least one type of biological analyte into the measuring chamber;
(c) Introduction of at least one type of fluorescence marker into the measuring chamber;
(c') Mixing of the functionalised particles, sample and fluorescence marker in the measuring chamber;
(c") Separation of the unbound fluorescence markers from the bonded fluorescence markers so that the bonded fluorescence markers are located in the separation region (205);
(d) Measurement of the fluorescence emissions of the unbound fluorescence markers in the detection region (106); and
(e) Determination of the quantity and/or concentration of the biological analyte or analytes.

4. The method according to claim 3, **characterised in that** steps (a), (b) and (c) occur sequentially or at least two steps occur simultaneously.

5. The method according to claim 3, **characterised in that** step (a) and/or step (c) are carried out before the remaining steps and with a large time interval relative thereto, wherein the time interval is greater than 12 hours.

6. The method according to any one of claims 1 to 5, wherein the method is a direct immunoassay, sandwich immunoassay, suppression or displacement immunoassay, competitive immuoassay and secondary immunoassay.

7. A microplate for carrying out the method according to any one of claims 1 to 6 in which at least one well is arranged as a measuring chamber (101), the measuring chamber comprising a topless or closed chamber which has side walls and the base of which is formed by a base of a device, wherein the device has an at least partially translucent structural element (103) which is designed as a protrusion which tapers upward, the cross section of which has any geometry, wherein the end of the structural element (304) which is facing away from the base of the device has a flat or convex shape and is provided such that no particles can settle there and the base of the measuring chamber, apart from the base area of the structural element (110), is opaque.

8. The microplate according to claim 7, **characterised in that** functionalised particles and/or fluorescence markers are located in one of the measuring chambers in dried form.

9. A kit for carrying out the method according to any one of claims 1 to 6 containing a microplate according to claim 7, at least one type of functionalised particle, at least one type of fluorescence marker and a reaction buffer.

10. A method for the production of a microplate according to claim 7, comprising the following steps:
(x) replacing the bottom of a microplate by a base having a structural elements which base preferably has as many structural elements as there are wells in the microplate;
(y) connecting the base to the microplate; and
(z) applying an opaque layer to the underside of the base, wherein the base area of the structural element (101) is kept free.

## Revendications

1. Procédé destiné à la détermination quantitative d'analytes biologiques dans une solution aqueuse en présence d'une ou de plusieurs surfaces fonctionnalisées, la solution aqueuse contenant au moins un type d'analytes biologiques et au moins un type de marqueurs fluorescents, **caractérisé en ce que**, pour déterminer la quantité et/ou la concentration de l'analyte ou des analytes biologiques, l'émission de fluorescence des marqueurs fluorescents non liés est mesurée, et dans lequel des particules fonctionnalisées de polymère ou d'un mélange de polymères sont utilisées en tant que surfaces fonctionnalisées, particules qui présentent un diamètre moyen dans une plage de 20 µm à 200 µm et sur la surface desquelles des molécules de capture sont présentes qui forment une liaison avec l'analyte ou les analytes biologiques et/ou avec le ou les marqueurs fluorescents en utilisant une chambre de mesure (101), comprenant une chambre ouverte ou fermée en haut qui est dotée de parois latérales et dont la base est formée d'une base d'un dispositif, ledit dispositif présentant un élément structurel au moins partiellement transparent (103) qui est conçu comme une surélévation effilée vers le haut dont la section transversale présente une géométrie quelconque, l'extrémité de l'élément structurel (304) détournée de la base du dispositif ayant une forme plate ou convexe et étant tel qu'aucune particule ne s'y dépose et le fond de la chambre de mesure étant opaque à l'exception d'une zone (110) située en dessous de l'élément structurel.

2. Procédé selon la revendication 1, **caractérisé en ce que** l'élément structurel est transparent et en forme de dôme, cône, cône coupé, pyramide, pyramide coupée dont la surface de base est polygonale, poly étant un chiffre compris entre 3 et 10.

3. Procédé selon la revendication 1 ou 2, comprenant les étapes suivantes :
(a) Introduction d'au moins un type de particules fonctionnalisées dans la chambre de mesure ;
(b) Introduction d'un échantillon comprenant au moins un type d'analyte biologique dans la chambre de mesure ;
(c) Introduction d'au moins un type de marqueurs fluorescents dans la chambre de mesure ;
(c') Mélange des particules fonctionnalisées, de l'échantillon et des marqueurs fluorescents dans la chambre de mesure ;
(c") Séparation des marqueurs fluorescents non liés des marqueurs fluorescents liés de telle sorte que les marqueurs fluorescents liés se trouvent dans la zone de séparation (205) ;
(d) Mesure de l'émission de fluorescence des marqueurs fluorescents non liés dans la zone de détection (106) ; et
(e) Détermination de la quantité et/ou de la concentration de l'analyte ou des analytes biologiques.

4. Procédé selon la revendication 3, **caractérisé en ce que** les étapes (a), (b) et (c) sont réalisées de manière séquentielle ou au moins deux des étapes sont réalisées simultanément.

5. Procédé selon la revendication 3, **caractérisé en ce que** l'étape (a) et/ou l'étape (c) ont lieu avant et à un intervalle de temps par rapport aux étapes restantes, l'intervalle de temps étant supérieur à 12 heures.

6. Procédé selon l'une quelconque des revendications 1 à 5, le procédé étant un test immunologique direct, un test immunologique sandwich, un test immunologique de déplacement, un test immunologique compétitif et un test immunologique secondaire.

7. Plaque microtitre pour la mise en œuvre du procédé selon l'une quelconque des revendications 1 à 6 dans laquelle au moins un puits est conçue comme une chambre de mesure (101), la chambre de mesure comprenant une chambre ouverte ou fermée en haut qui est dotée de parois latérales et dont la base est formée de la base d'un dispositif, ledit dispositif présentant un élément structurel au moins partiellement transparent (103) qui est conçu comme une surélévation effilée vers le haut dont la section transversale présente une géométrie quelconque, l'extrémité de l'élément structurel (304) détournée de la base du dispositif ayant une forme plate ou convexe et étant tel qu'aucune particule ne s'y dépose et le fond de la chambre de mesure étant opaque à l'exception d'une zone (110) située en dessous de l'élément structurel.

8. Plaque microtitre selon la revendication 7, **caractérisée en ce que** des particules fonctionnalisées et/ou marqueurs fluorescents se trouvent à l'état sec dans au moins une des chambres de mesure.

9. Kit pour la mise en œuvre du procédé selon l'une quelconque des revendications 1 à 6 comprenant une plaque microtitre selon la revendication 7, au moins un type de particules fonctionnalisées, au moins un type de marqueurs fluorescents et un tampon de réaction.

10. Procédé destiné à la fabrication d'une plaque microtitre selon la revendication 7, comprenant les étapes suivantes :
(x) Remplacement du fond d'une plaque microtitre par une base présentant des éléments structurels, base qui présente de préférence autant d'éléments structurels qu'il y a de puits dans la plaque microtitre ;
(y) Jonction de la base avec la plaque microtitre ; et
(z) Application d'une couche opaque sur le côté inférieur de la base, la zone située en dessous de l'élément structurel, à savoir la surface de base de l'élément structurel (110), n'étant pas recouverte.
